(19) 〔Europäisches Patentamt / European Patent Office / Office européen des brevets〕

(11) **EP 4 095 626 B1**

(12) **EUROPEAN PATENT SPECIFICATION**

(45) Date of publication and mention
of the grant of the patent:
**06.05.2026 Bulletin 2026/19**

(21) Application number: **21179569.5**

(22) Date of filing: **15.06.2021**

(51) International Patent Classification (IPC):
$C12M\ 1/00$ (2006.01)    $C12M\ 1/36$ (2006.01)
$G05B\ 15/00$ (2006.01)    $G06N\ 3/00$ (2023.01)
$G16B\ 40/00$ (2019.01)    $G16C\ 20/10$ (2019.01)

(52) Cooperative Patent Classification (CPC):
**C12N 13/00; C12M 41/48; G06N 7/01; G06N 20/00**

(54) **METHOD FOR INDUSTRIAL BIOREACTOR OPTIMIZED WITH MUTUALLY DEPENDENT, COUPLED PROCESS CONTROL LOOPS**

VERFAHREN FÜR INDUSTRIELLEN BIOREAKTOR OPTIMIERT MIT VONEINANDER ABHÄNGIGEN, GEKOPPELTEN PROZESSREGELKREISEN

PROCÉDÉ POUR L'UTILISATION DE BIORÉACTEUR INDUSTRIEL OPTIMISÉ AVEC DES BOUCLES DE COMMANDE DE PROCESSUS COUPLÉES MUTUELLEMENT DÉPENDANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2021 CH 6002021**

(43) Date of publication of application:
**30.11.2022 Bulletin 2022/48**

(73) Proprietor: **Bühler AG**
**9240 Uzwil (CH)**

(72) Inventor: **BUCHMANN, Leandro**
**8408 Winterthur (CH)**

(74) Representative: **Leimgruber, Fabian Alfred Rupert**
**ThomannFischer**
**Wettsteinplatz 7**
**4005 Basel (CH)**

(56) References cited:
**EP-A1- 3 819 367**    **CN-A- 105 543 085**
**US-B1- 10 751 715**    **US-B2- 10 336 978**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates generally to the industrial production of biomass, and in particular to industrial processes for generating biomass by providing a controlled cultivation process for cell cultures, components of cells or metabolic products of the cell cultures using bioreactors. Even more particular, the present invention relates to industrial processes for generating biomass by cell cultivation and treatment within bioreactors e.g. by applying a pulse-regulated electric field potential.

### Background of the invention

### (i) Bioreactors

Bioreactors (in the context of microbial or enzymatic conversion of organic substances into acid, gases or alcohol also referred to as fermenters), typically are containers in which certain microorganisms, cells or small plants are cultivated (fermented) under applied, technically controlled conditions. The operation of a bioreactor is thus an application of the field of biotechnology that uses or harnesses biological processes (bioconversion, biocatalysis) in technical facilities. Important factors, characteristics and/or operating parameters that are controllable or steerable in most bioreactors, are, for example, the composition of the nutrient medium (also nutrient solution or substrate), oxygen supply, temperature, pH value, sterility and others. The purpose of cultivation in a bioreactor can be the controlled recovery of the cells or components of the cells or the recovery of metabolic products or the controlled metabolism of cell cultures as such. The cells, components of cells or the metabolic products can be used, for example, as active ingredients in the pharmaceutical industry, as basic chemicals in the chemical industry, or in the context of cell cultivation, for example, in the cultured food production, as e.g. cultured meet production or fermented food, e.g. tempeh, miso, koji, and soy sauce etc., or in the medical applications in organ or tissue replacement treatments etc.. The degradation of chemical compounds can also take place in bioreactors, as in the treatment of wastewater in sewage treatment plants. The production of beer, wine and other products that have been produced historically also takes place in bioreactors (fermenters). Today, a wide variety of organisms are cultivated in bioreactors for different purposes. Several reactor variants are available in different designs. Examples are stirred tank reactors made of metal, which can have a volume of a few to thousands of liters and are filled with nutrient solution. However, widely differing variants, such as fixed-bed reactors, photobioreactors etc. are also used.

The main purpose of bioreactors are to deliver the highest possible product yields. This is achieved in particular by creating optimal conditions for the organism or cells used in the specific case. The conditions in the bioreactor are adapted to various parameters that prevail in the natural habitat or the cultivated organisms or the natural environment of the cultivated cells. Typically, cultivation process parameters as the type and concentration of nutrients, temperature, oxygen content, pH value, etc. are important or critical to achieve an optimized cultivation. The control of the parameters is realized using technical devices as e.g. agitators or other devices necessary to ensure a homogeneous adjustment of these parameters over the reactor chamber. In addition to the requirements of the organisms or cells, other technical, organizational, and economic factors often are to be considered that influence the choice of operating parameters. Examples can be, inter alia, the prevention of foam formation and the choice of either a continuous or a batch mode of operation. Using probes or sensors, many of these parameters can e.g. be measured directly in the nutrient medium or in the exhaust air. In addition, the course of the process can usually also be assessed or monitored via these parameters. The cell density can be determined by measuring the absorbance (optical density), which in turn allows conclusions to be drawn about the product quantity. An alternative is often the measurement of the concentration of a characteristic chemical compound, e.g. the increase in concentration of a metabolic product or the decrease in substrate concentration, for example, by optical sensors or other appropriate sensors. At the beginning of a cultivation or fermentation process, typically a small amount of the microorganisms or cells to be cultivated and obtained from a pre-cultivation are added to the culture medium. This amount is called inoculum, and the process is often referred to as inoculation. The suspension obtained from the cultivation process is prepared in several process steps during so-called downstream processing.

The nutrient medium must provide the organisms/cells with all the nutrients they need for growth. These include e.g. main nutrients (macronutrients) required in larger quantities, such as carbon, nitrogen and phosphorus. Further, various trace elements (micronutrients) can be needed. Depending on the organisms/cells to be cultivated, other compounds can be required that cannot be synthesized by the organisms/cells itself (e.g. vitamins, essential amino acids, etc.). Energy-providing compounds, such as sugar glucose, can also be necessary (except in phototrophic organisms). Organisms and cells have typically an optimum temperature at which they reproduce best. Exceeding this temperature can lead to irreversible damage through denaturation of the proteins, falling below it can lead to lower metabolic rates and thus to longer process durations. Temperature control can e.g. be realized by the control of applied heating and cooling circuits. When the bioreactor is started up, the entire reactor contents are heated or warmed to operating temperature. In some cases, the cultivated organisms or cells generate so much waste heat through their metabolism that only the cooling circuit is active above a certain cell concentration. A heat exchanger can be integrated into this circuit, or the energy-bearing medium can be fed in directly. In this case, the only heat exchange surfaces available to the reaction chamber are usually the double vessel wall, or in other cases also built-in cooling registers.

Depending on the organisms or cells cultivated and/or the product, cultivation (fermentation) processes can be carried out aerobically (in an oxygen-containing atmosphere) or anaerobically (without oxygen). Since oxygen is poorly soluble in water, it can be difficult to adequately supply aerobic approaches. For example, the oxygen solubility in a fermentation medium with a temperature of 37 °C is 3-5 mg/L. The oxygen partial pressure in the reactor can be regulated by different methods, as e.g. (i) changing the gas flow rate, (ii) changing the stirrer speed, (iii) changing the stirrer tool geometry, (iv) changing the gas mixture composition, or (v) changing the head pressure (however, this also increases the solubility of other gases, for example carbon dioxide). In the case of anaerobic organisms, on the other hand, oxygen supply typically should be avoided as it can have a toxic effect. In anaerobic approaches with anaerobic organisms, oxygen supply can e.g. enable undesired aerobic reactions, which can reduce the process yield. The cultured organisms or cells usually have a limited pH-value tolerance range with a pH optimum. The pH can e.g. be controlled with pumps coupled or linked to a pH-sensor via a control steering and/or a Programmable Logic Controller (PLC), where the pump pumps, if required e.g. phosphoric acid ($H_3PO_4$), hydrochloric acid (HCl), or sodium hydroxide solution (NaOH) etc., into the bioreactor to acidify or raise the pH-value. In certain cases, a certain pH-value can also be achieved by adapting the rate of feeding with substrate. To reach a certain homogenization, bioreactors can further comprise stirring devices, such as an agitator or gas injection, through which the medium is circulated. This is used to ensures a possibly homogeneous setting of various parameters throughout the reactor and thus a more uniform process flow. The mode of operation of a bioreactor can typically be distinguished between: (i) *batch* operation: filling of the reactor, no addition or removal during the cultivation/-fermentation process, simple control, contamination unlikely; (ii) *fed-batch* operation: similar to batch operation; however, e.g. substrate addition during the process, as an initially high substrate concentration can be inhibiting; and (iii) *continuous operation in chemostat* bioreactor: uninterrupted operation by substrate addition and product removal, complex control, contaminations problematic, but expensive and complex downstream processes can also be carried out continuously, and thus more optimally utilized.

The operating parameters to be maintained can be very different for each organism or cell type for technical or other reasons. Therefore, for particular uses, an appropriate bioreactor must often be designed, or specific types of bioreactors may be used in which the various parameters can be controlled within a wide parameter value range or window so that the reactor can be used for different cultivations of different organisms or cells. A common type is the gasable stirred tank reactor in different variants (material, size, etc.). In the prior art, a classifying distinction can be made according to the type of reactor design comprising e.g.: (i) Stirred tank reactor: widespread type; the liquid phase is circulated by an agitator; gassing is performed as required; (ii) fixed-bed reactor: the reactor is filled with a solid, porous matrix on which the organisms (or enzymes) can be immobilized; the organisms thus remain in the reactor instead of being flushed out with the medium, so that the growth of the organisms is a less limiting factor (or the enzyme requirement is reduced); (iii) trickling stream reactor (trickling filter): a fixed bed is sprinkled with liquid (e.g. wastewater to be treated); the degrading organisms sit on the surface; the air (oxygen) required for degradation is introduced in countercurrent flow; (iv) photobioreactor (algae reactor, hydrogen bioreactor): for the cultivation of organisms (algae, plant (cells)) that carry out photosynthesis; the reactor is made of glass so that the necessary light reaches the organisms; the utilization of light can e.g. be optimized by plate- or tube-shaped reactors; (v) tubular reactor: in tubular reactors a plug flow can be created; and (vi) membrane bioreactor: a reactor in which (depending on the application) the reaction product, biomass or purified water can be permanently separated by membranes. Applications for this include wastewater treatment (MBR), lactic acid recovery, and pharmaceutical products. Finally, the processes in bioreactors can technically be simulated e.g. by parameter processing by means of implemented reaction kinetics, although the simulation must take into account the specifics of biological processes (e.g., Michaelis-Menten theory, Monod kinetics, enzyme kinetics, enzyme inhibition, etc.).

In industrial processes and device settings, as e.g. bioreactors, control systems are used to monitor, control, steer and signal the bioreactors, facilities, plants or other equipment, and operation/processes of industrial or chemical/biochemical processes, as the cultivation or treatment processes within the bioreactors. Typically, the system that performs the controlling and monitoring uses field devices distributed at key locations in the industrial process, e.g. within the bioreactor, coupled to control circuitry by a process control loop. The term "field device" refers to any device that performs a function in a distributed control or process monitoring system, including all devices used in the measurement, as e.g. sensors and measuring devices, control, monitoring and signaling of industrial processes and processing facilities. Each field device can e.g. comprise communication means, and circuitry used for communicating, in particular wired or wireless communication, with a process controller, other field devices, or other circuitry, over the process control loop. In some installations, the process control loop is also used to deliver a regulated current and/or voltage to the field device for powering the field device. The process control loop also carries data, either in an analog or digital format. Typically, field devices are used to sense or control process variables in an industrial process and/or in specific installations, if required, to monitor the local environment of the field device.

In summary, bioreactors, sensors, and methods for measuring relevant parameters are known (see for example also EP 2725095 B1, WO 2016092281 A1, CN 105044038 A, US 10751715 B1). However, further improvements are desirable, especially for applications in cell culture and/or microbiology controlling and steering the performance of the cultivation process.

### (ii) Cell metabolism and lipid bilayer membranes

In the light of the above-discussed, it is clear, that understanding the cell characteristics and the cell mechanisms interacting physically with the surrounding area (extracellular medium) and the area's environmental conditions is fundamental for bioreactors providing an industrial and scalable process e.g. in order to generate biomass by cell cultivation and treatment. Basis of cell reproduction is metabolism which can be defined as the set of life-sustaining chemical reactions in organisms or cells. The three core elements of metabolism are: (i) the conversion of nutrient to energy to run cellular processes; (ii) the conversion of nutrient to building blocks for proteins, lipids, nucleic acids, and some carbohydrates; and (iii) the elimination of metabolic waste products. These enzyme-catalyzed reactions allow organisms to grow and cells to reproduce, maintain the cell structures, and respond to their environments. The word metabolism can also refer to the sum of all chemical reactions that occur in living organisms or cells, including the transport of substances into and between different cells, in which case the above described set of reactions within the cells is called intermediary or intermediate metabolism.

In particular for the intermediary or intermediate metabolism, lipid membranes serve as effective barriers allowing cells to maintain internal composition differing from that of extracellular medium. Membrane permeation, both natural and artificial, can take place via appearance of transversal pores. For plasma membranes, lipid bilayer constitutes a major structural component. Said lipid bilayer (or phospholipid bilayer) is a thin polar membrane made of two layers of lipid molecules. These membranes are flat sheets that form a continuous barrier around all cells. The cell membranes of almost all organisms and various viruses are made of a lipid bilayer, as are the nuclear membrane surrounding the cell nucleus, and membranes of the membrane-bound organelles in the cell. The lipid bilayer is the barrier that keeps ions, proteins and other molecules where they are needed and prevents them from diffusing into areas where they should not be. Lipid bilayers are ideally suited to this role, even though they are only a few nanometers in width, because they are impermeable to most water-soluble (hydrophilic) molecules. Bilayers are particularly impermeable to ions, which allows cells to regulate salt concentrations and pH by transporting ions across their membranes using proteins called ion pumps. The amphiphilic nature of the lipid molecules, i.e. molecules possessing both hydrophilic (water-loving, polar) and lipophilic (fat-loving) properties, thus containing both polar and hydrophobic parts, determines low permeability of lipid bilayers for broad range of substances and thus allows the membranes to perform barrier function effectively in the cells. Artificial permeabilization of plasma membranes is used for various bioengineering and medical purposes. There are two alternative mechanisms of penetration through the membranes: small individual molecules can cross membranes, presumably through local defects of lipid packaging, and water-filled pores through the entire membrane can be formed, enabling non-specific transfer of various polar substances. The present invention mostly relies on the mechanisms of formation of transverse pores in lipid bilayer membranes.

Thus, understanding of the barrier properties of biological membranes is fundamental for controlling metabolism in the bioreactor. In the prior art, investigable model systems are developed, such as artificial liposomes or vesicles, which mimic the geometry and size of the cell membrane but are devoid of ion channels and the multitude of other embedded components, commonly present in the cell membrane. The simplest model of a cell membrane patch is a planar lipid bilayer. The chemical composition of the planar lipid bilayer can be chosen in advance and is therefore well defined. However, to mimic a non-curved fragment of the cell membrane, the planar lipid bilayer should separate two electrolytes. Therefore, the planar lipid bilayer is usually vertically formed across a small aperture in a hydrophobic partition that separates two compartments filled with electrolytes. Visual observation of the planar lipid bilayer in such an experimental system is limited. However, electrodes immersed in the electrolyte permit measurements of electric parameters of the planar lipid bilayer. As a planar lipid bilayer can be electrically considered as a non-perfect capacitor, the capacitance C of an ideal capacitor and the resistance R of a resistor in parallel both describe planar lipid bilayer's characteristic. Voltage-controlled and current-controlled methods enable observing electrical properties of the planar lipid bilayer and its structural changes that are reflected in the electrical characteristics. In the prior art, the capacitance C is considered as the most suitable measuring parameter for probing the stability and proper formation of planar lipid bilayer and consequently, C is almost always measured for each planar lipid bilayer investigated with electrical methods, even when other properties are technically in the focus. An exposure of planar lipid bilayer to external electrical stimulus and formation of transmembrane voltage alter both electrical parameters of planar lipid bilayer system, R and C. Molecular dynamics (MD) simulations have shown that small so-called water fingers are formed on both sides of the planar lipid bilayer in the presence of transmembrane voltage and create water or hydrophobic pores. The lipids adjacent to the water molecules inside the pores start reorienting their polar headgroups toward water molecules and stabilizing the pores into hydrophilic state, which allows more water and ions to enter the pores. These conductive paths reduce R of the planar lipid bilayer. At low transmembrane voltages, when pores are not created yet, a capacitance increase can be observed. An electrostrictive thinning of the planar lipid bilayer can be assumed as the mechanism responsible for this phenomenon. The capacitance of the planar lipid bilayer depends on applied voltage U according to the relation

$$C(U) = C_0[1 + \alpha U^2]$$

where $C_0$ is the planar lipid bilayer capacitance at zero transmembrane voltage, and $\alpha$ is the proportionality coefficient. Experiments revealed that its value is typically around 0.02 $V^{-2}$, while using numerical simulation models, it was assessed to be in the range of 0.053-0.082 $V^{-2}$. However, at higher transmembrane voltages, appearance of water and hydrophilic pores leads to reduction of the planar lipid bilayer capacitance, due to significant differences in dielectric constant values of lipid bilayer and water. A long-lasting exposure to strong electric stimulus causes irreversible damage to a planar lipid bilayer. Electrical measurements permit determination of the breakdown voltage value $U_{br}$.

### (iii) Electroporation: hydrophobic pores and hydrophobic defects

Hydrophobic pores formed in planar lipid bilayer can e.g. be modeled by the known Abidor's model of electroporation, where water pores can be successfully captured by molecular dynamic simulation. Since such water pores are very small transient structures, they cannot be directly observed. However, the experimentally observed increase in the variance of membrane current, that is attributed to small current fluctuations due to membrane capacitance changes, can be connected to the number of hydrophobic pores in the membrane during the phase transition. Even experimental information about conducting hydrophilic pores and their characteristics comes only from indirect measurements, like current fluctuations measurements, voltage fluctuations measurements, or observing voltage drops. Newer, improved theories on the pore formation allow capturing continuous trajectories of pore formation, both in the absence and presence of stress conditions. Those theories may explain the complex behavior of pores during their formation under lateral tension, even in various loading rates regimes that can be studied in giant unilamellar vesicles (GUV) aspiration experiments. Namely, at low loading rates, GUV membrane ruptured at small tensions and, according to theory, membrane breakdown is governed by the pore expansion. On the other hand, at high loading rates, GUV membrane rupture occurs at high values of tensions and is theoretically limited by water pore formation, which is lipid type (curvature) dependent. Akimov's theory predicts that the planar lipid bilayer, exposed to the electric field, behaves similarly as in the presence of lateral tension. Experimentally, it can be shown that an increase in the permeability of the lipid bilayer of biological membranes, manifested in the form of discrete conductivity jumps at electrical measurements, in the leakage of the contents of liposomes and in the enhancement of flip-flop, occurs under the action of an electric field and membrane tension, close to chain melting transitions as well as at the addition of detergents. It seems likely that the molecular mechanisms for increasing the permeability of the bilayer in all of these cases have much in common. In particular, it seems plausible to associate the increase in the lipid bilayer permeability with the formation of hypothetical hydrophobic defects and hydrophilic pores. Although available experimental methods do not allow direct studies of these structures, the occurrence of current fluctuations in flat bilayers, leakage of liposomes, and flip-flop enhancement can serve as indirect evidence for the existence of hydrophilic pores in the bilayer.

To understand the formation of hydrophilic pores, it can be assumed that a hydrophilic pore is formed in a originally intact bilayer through an intermediate state referred to as hydrophobic defect. There are evidences from electroporation experiments for the existence of small, metastable "pre-pores", referred as hydrophobic defects, in the a stage of hydrophilic pore formation process. However, the structure, characteristics and process of occurrence of through hydrophobic pores as well as the molecular mechanisms of the formation of primary hydrophobic defects are still unclear, and not explained e.g. by using the methods of molecular dynamics. Hydrophobic defects can occur in an intact membrane as a result of thermal fluctuations. However, they do not lead to macroscopic consequences, since the frequency of their appearance is small. The experimentally observed increase in membrane permeability may be associated with an increase in the frequency of occurrence and the number of hydrophobic defects under the action of a stimulus (electric field, thermodynamic changes during a phase transition, etc.). For example, the occurrence of a sufficiently large number of hypothetical hydrophobic defects during a phase transition can be associated with an increase in area fluctuations. One difficulty of describing this stage of pore formation and evolution lies in the lack of experimental data associated with hydrophobic pores. At the present, pre-pores (hydrophobic defects) are only indirectly detected by observing the effects of "memory" in membranes and flickering of conducting events during the lifetime of the pre-pores. Thus, experimental data associated with the existence of hydrophobic pores in the lipid bilayers and allowing to measure their number and cause of occurrence are of a fundamental interest. As a possible approach, the Smoluchowski equation with an energy profile of a special type and an assumed hydrophobic pore source term can e.g. be used to capture the process of hydrophilic pore formation in a lipid bilayer at the gel-liquid phase transition. The source term reflected the occurrence of molecule packing defects in a lipid bilayer at phase transition. That permitted to model the temporal sequences of current impulses occurring at phase transitions with various thermo-dynamic characteristics. In the classical theory of electroporation, the hydrophobic pores are presented in the form of water columns in contact with the hydrophobic lipid tails. Such a pore does not allow the transit of ions but can change the capacitance of membrane by changing the dielectric permittivity. As a hydrophobic pore opens or closes under a fixed voltage regime, current fluctuations must thus be induced, with the current mean being equal to zero. For modelling the increase in current variance through membranes can be measured, the temperature of which decreases, approaching the phase transition temperature. The modelling then connects the number of hydrophobic pores and the variance of membrane current. The number of hydrophobic pores as obtained from measurements can, thus, be compared with the theoretical value as provided using the Smoluchowski equation with a source of hydrophobic pores reflecting the occurrence of packing defects in the lipid bilayer at phase transition. Based on this model, estimates of the number of hydrophobic pores in the membrane during the phase transition can be obtained.

### (iv) Nanosecond pulsed electric field application (nsPEF)

The last few decades have seen an increased interest in the application of pulsed electric fields (EPS), which has been particularly applied in the medical and biotechnology sectors. They have been shown to have broad applications in the food processing industry, for cryopreservation of cell lines, enhancing gene or drug uptake by cells, and induce cell death through necrotic or apoptotic pathways. Understanding how electric fields influence cell behavior is important when considering how to technically improve or expand current treatment options. Above, we already discussed the cell and its intracellular contents, and more specifically the membranes that surround them, where electric fields are expected to interact.

In the technical field of bioengineering, electroporation (also electropermeabilization) is defined as the exposure of biological materials (cell suspensions, tissues) to high voltage electric pulses provoking cell membranes to become more permeable to molecules that otherwise cannot cross them. Unseen the technical and theoretical problems in modelling and understanding the process, electroporation is nowadays widely used as a basis for applications in biotechnology and medicine such as electrochemotherapy, gene electrotransfer, tissue ablation, extraction of various compounds, and microbial inactivation in food preservation. In the application of electroporation, it could be shown that shorter electric pulses of nanosecond duration (nsEP: nanosecond Electric Pulses; also referred as nsPEF: nanosecond Pulsed Electric Field) have more profound effects on the cell interior than longer pulses of micro- and millisecond duration, and thus nsPEF can be used as a tool for intracellular manipulation without any chemical intervention. With the development of new pulse generators that produced ultrashort pulses of very high electric fields of several tens of MV/m (Millivolt/meter (electric field strength)), the effects can be shown on cells, the nanosecond electric pulses affecting cell organelles, increase intracellular calcium, and provoke apoptosis and stress responses. Further, also the plasma membranes are affected. The pores produced by nsPEF are small, of nanometer scale, and are thus also referred to as "nanopores". Cells exposed to nsPEF exhibit membrane permeability to both propidium (PI) and trypan blue (TB), classical indicators of membrane permeabilization, but detecting influx of these dyes and other small molecules after nanosecond pulse exposure requires methods with greater sensitivity than those used for longer pulses. However, using different cells, pulse parameters, exposure configuration and detection methods can lead to different results. Thus, the industrial use of nsPEF in bioreactors is not trivial and may have complex dependences the technical setup of the bioreactor and the used operational parameters.

The application of nsPEF to cells can e.g. be classified into distinct frequency regions, as: (A) 1-10 ns, (B) 11-100 ns and (C) 101-999 ns. With the use of nanosecond pulses, as pulses shorten, more intracellular effects and less effects on plasma membrane are typically expected. The first category (A) includes very short pulses, 1-10 ns, with rise times of a few ns (mostly shorter than the electrolyte relaxation time). In this regime the dielectric properties of the membrane and the intracellular and extracellular media dominate pulsed electric field effects on membranes, and the Maxwell-Wagner polarization of the membrane by migration of mobile charges is less important than it is for longer pulses. Moreover, proportionally greater effects can be expected on intracellular membranes with 1-10 ns pulses than with longer pulses. In the second category (B), the pulse duration is less than the charging time of the plasma membrane. The third category (C) includes nsPEF with durations longer than the plasma membrane charging time. It is a demand in the technical field, to provide an optimized bioreactor and bioreactor process, inter alia, controlling the role of pulse duration in regard to the accompanying measurable effects of nsPEF on biological cells, as eukaryotic human, animal, and plant cell cultivation in bioreactors. Such an optimized bioreactor and bioreactor cultivation process has to be enabled to react on various conditions, e.g. since the occurrence of changes in the plasma membrane can significantly depend on the nsPEF pulse duration, and/or the occurrence of intracellular effects can significantly depend on the nsPEF pulse duration, where PM effects may be greater with longer pulses and intracellular effects may be greater with shorter pulses.

Further for the technical setup, during the electroporation process biological cells are exposed to pulsed electric fields with specific electrical parameters, as amplitude, duration, shape, number of pulses and pulse repetition rate. The duration of the pulse can be specified as the full width at half maximum (FWHM) and a description of the pulse shape can be enhanced with rise and fall times of the pulse. In order to exactly specify the technical method and thus to enable the reproduction of the cultivation and treatment process within the bioreactor, it is typically necessary to exactly determine these electrical parameters. Some electrical parameters such as number of pulses and pulse repetition rate are relatively easy to capture. Other electrical parameters are more difficult to determine, because it is difficult to measure the time course and distribution of the nanosecond electric field within the exposure configuration during the delivery in the bioreactor. In the prior art on the electroporation of biological cells by nsPEF there is a need for the precise determination method and optimized control of the electric field to which biological cells are exposed in bioreactor and the cultivation and/or treatment process.

There are several factors known influencing the outcome following membrane exposure to an electric field. Generally it can be assumed that the initial step in membrane breakdown following PEF exposure is the magnitude of the induced transmembrane potential. Thus, the electric field strength can play an important role in this process. Finding the threshold for this response has been the subject of many bioreactor systems seeking to exploit this consequence of PEF application. Achieving this threshold; however, is highly dependent on the duration of the pulse delivered, such that eliciting a response using a shorter pulse would require a stronger electric field than the same response using a longer pulse. For example, using pulses ranging from 1ns to 100ms to stimulate specific muscle cells, an applied voltage of 4.5 kV may be required for a 1.8ns pulse to elicit a similar response to a 100ms pulse delivered at 0.02 kV. In addition to the electric field intensity and pulse duration, other important features are; the number of pulses and the frequency at which they are delivered.

Another feature becomes evident when applying different pulse shapes. For example, using $\mu$sPEFs with the electric field intensity required to permeabilize 50 % of the cells, this may be reduced by 20 % when symmetrical bipolar pulses are used. The reason may lie in the fact that i) the polar asymmetry following a unipolar pulse is counterbalanced when a bipolar pulse is delivered and ii) bipolar pulses increase the odds that a non-spherical cell are permeabilized. However, in contrast to this, there may also occur a dampened effect when bipolar nanosecond pulses are used. For example, the addition of a second phase (of opposite polarity) may cancel the effect of the initial monopolar phase in both intracellular calcium mobilization and cell survival. A similar dampening effect can be observed for calcium uptake as well as measures of plasma membrane integrity when comparing bipolar effects to monopolar effects. Unseen the existing models for capturing the differences between $\mu$s and ns bipolar pulses, it can be stated that the duration of the pulse is critical and is based on a biological constant known as the membrane charging constant

As discussed above, the induced transmembrane potential can be assumed to be due to the accumulation of ions along the cells membrane. This process requires time, and is referred to as the charging time, or charging constant. Although it varies slightly between cells, the charging time is on the order of a few hundred nanoseconds. Pulse durations that are longer than the charging constant will have very different effects those that are shorter. In this application, pulses that are longer than the charging time will be referred to as pulsed electric fields (or PEF), and those that are shorter will be referred to as nanosecond pulsed electric fields (or nsPEF).

At longer durations, in the micro- or millisecond range, PEF have been shown to be effective at delivering proteins, drugs, and DNA across the cell membrane. By adjusting the number, frequency and intensity of the pulses one can reversibly or irreversibly permeabilize the plasma membrane. In the prior art, irreversible electropermeabilization (IRE) has been mainly applied as an electrical treatment for tumors. With IRE, the cell is unable to repair the damage imposed by the electric field. Contrary to IRE, reversible electropermeabilization utilizes the ability of the membrane to repair itself as an advantage. Of the two, reversible electropermeabilization has been most thoroughly investigated, and offers a greater number of potentials. Gene electrotransfer (GET) is one example of such application, where DNA is introduced into the cell and makes it possible to regulate the production of a desired transgene. In the medical field, vast number of disorders that can potentially be treated using reversible electropermeabilization systems, including Parkinson's disease, HIV/AIDS and cancer etc.. Electrochemotherapy (ECT) is another technical application for reversible electropermeabilization, where chemotherapeutics (or high concentrations of calcium) can be injected systemically or locally followed by electropulsation of the tissue.

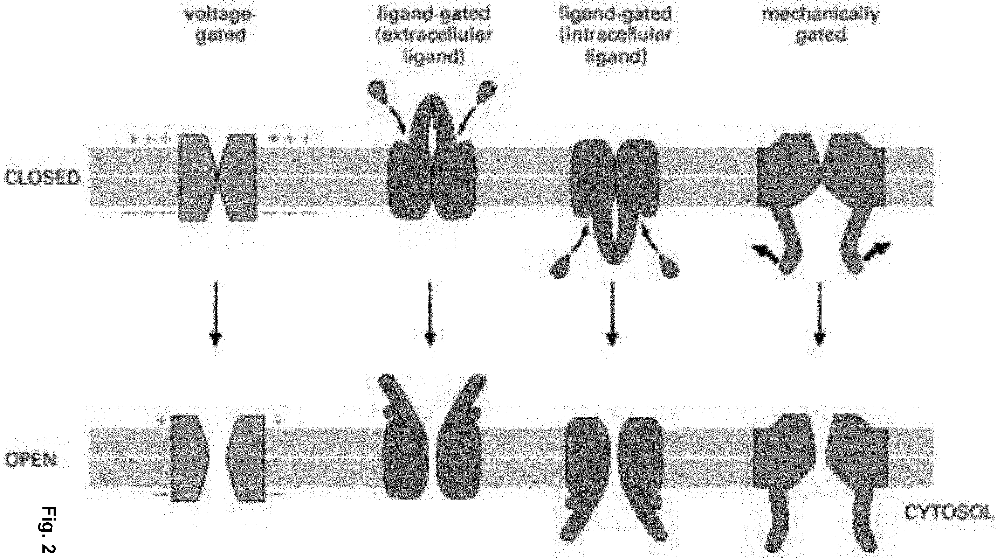

In nsPEF, when pulse durations shorter than the charging constant are applied, the plasma membrane no longer shields the intracellular environment, and very different effects typically may occur. Effects such as mitochondrial depolarization, caspase activation and nuclear condensation; all effects typical of cell death by apoptosis, can be observed. Additional effects such as cytoskeleton disruptions, including microtubule and actin assembly have also been observed. Whereas intracellular effects are specific to nsPEF, there is a lot of evidence suggesting additional effects occur on the plasma membrane. For example, membrane permeability was found to be enhanced following nsPEF exposure. It can be assumed that these ultra-short fields may exert effects directly on transmembrane protein channels in the plasma membrane. In prior art, experimental evidence exists that supports this, where nanosecond pulses have resulted in direct activation of voltage-gated channels (cf. figure 2).

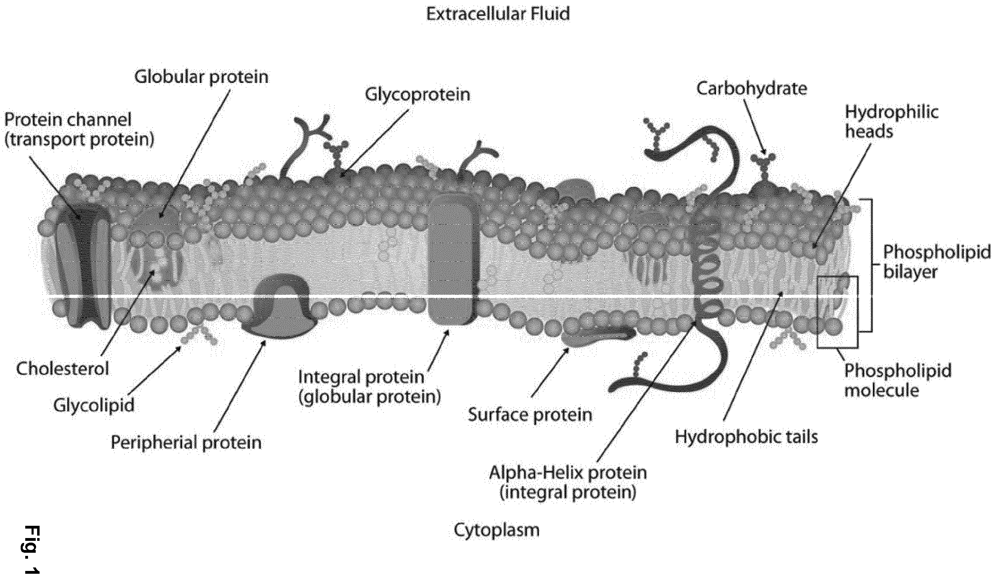

Disregarding the application in the medical field, the application of pulsed electric fields (PEF) and nsPEF can be used in various areas of the food industry and bioprocess engineering. Here the objective is to influence the product cell structure and/or the metabolism of the cultivated cells. The cells can be plant or animal cells, e.g. those of a potato or meat, but also of microorganisms. The cells show differences in physical characteristics, as size and particularly in their composition. However, all cells are surrounded by a membrane with phospholipids as its main component (cf. figure 1). Also for the bioprocess engineering, e.g. in bioreactors, due to the properties of the phospholipids, the membrane can be seen as a non-conductor, and the cell therefore has a natural charge, already mentioned above as trans-membrane voltage. For PEF, the application of an external voltage, a charge accumulation and an increase in the potential is induced and an electrical compression is triggered. This results in the formation of a pore in the membrane, which is the above-discussed process of pore induction with PEF (electroporation). Also in bioprocess engineering, depending on the applied pulse characteristic (electric field strength, pulse shape and width, energy input), reversible or irreversible pores can be formed. Reversible pores are hydrophilic and close by themselves after a short time. With a higher intensity of the electric pulses and a longer treatment, the initially hydrophilic pores turn into hydrophobic pores that cannot be closed again. This causes permanent damage to the cell. For the microorganisms contained in the product, the loss of the boundary to the environment means the loss of viability. Plant cells, e.g. potato cells, loose their internal cell pressure (turgor) when subjected to the PEF treatment and the increase in the membrane permeability results in an easier mass transport, for example when extracting valuable ingredients. As such, PEF treatment applications can be used for a variety of different applications, as inactivation of microorganisms, the reduction of vegetative germs, inactivation of bacterial endospores, drying of products etc.

In addition to plant cells, also animal cells, e.g. meat cells, can be influenced by PEF or nsPEF treatment. The present invention, inter alia, relates to systems and methods for enhancing cultured meat production, such as livestock-autonomous meat production. The conceptual promises of "cultured meat" (e.g., animal-autonomous meat production by in vitro cell culture, tissue engineering, and food technology meth-ods) include increased production efficiency, reduced environmental impacts, expanded culinary application utility, enhanced nutritional value, cruelty-free production and improved food safety relative to conventionally produced meats. Technologies to date, however, have not advanced sufficiently to support scalable, economically sustainable production. The current laboratory-scale cultivation of prototype tissues has utilized primary animal components such as animal tissues and serum, thereby largely negating the advantages of animal-autonomous meat production. Hence, current methods fail to resolve the animal dependence from cultured meat production sufficiently to realize the conceptual promises of "cultured meat" and provide a commercially advantageous product. Therefore, there is a need to provide new and improved methods for scalable meat cultivation from a self-renewing source in vitro for dietary nutrition and other applications. Further, the technical challenges in manufacturing cultured meat products, unseen the need for a production of large quantities of cells, include producing products having sensory qualities appealing to the consumers, including visible appearance, texture, flavor and aroma. However, main challenge remains to scale-up the production processes in order to manufacture the meat products in large quantities suitable for human consumption. A particularly challenging mission is the direct production of a meat portion suitable for serving, rather than separate cultured meat aggregates or layers that need to be fused or connected in order to obtain a meat portion suitable for serving.

Attempts for producing large quantities of mammalian cells, and to adhering the cells to a substance have also been taken in the pharmaceutical industry, for example for producing stem cells for therapeutic use and for tissue and organ grafting. For examples the patent applications US 6,911,201 and US 2010/0233130 disclose methods of producing undifferentiated hemopoietic stem cells using a stationary phase plug-flow bioreactor. In some embodiments, the methods comprise seeding undifferentiated hemopoietic stem cells or progenitor cells into a stationary phase plug-flow bioreactor in which a three-dimensional stromal cell culture has been pre-established on a substrate in the form of a sheet, the substrate including a nonwoven fibrous matrix forming a physiologically acceptable three-dimensional network of fibers. The document WO 2008/152640 discloses a method of transplanting the three-dimensional stromal cell culture comprising hematopoietic stem cells into a recipient. Another example can be given by US 9,127,242 disclosing a single-use, single or multiple tissue, organ, and graft bioreactor and environmental control system.

In summary, nsPEF induced growth stimulation by an electric field in bioreactors is known in the prior art, in particular there are documented effects in fungi, soy, microalgae, and other cells. However, controlled and reproducible growth stimulation was currently not possible. Electric fields and PEF may have precise stimulation windows, but controllable and reliable growth and/or compound stimulation has only been achieved under nsPEF conditions. However, since growth stimulation based on nsPEF processing yielded promising results using batch systems, nsPEF has the potential to increase growth as well as specific cellular compounds while maintaining techno-functional properties of the remaining compounds, if suitable systems are provided. Moreover, nsPEF-based growth/cellular compound stimulation has the potential to increase resource efficiency, economic viability, and the affordability of the derived products, thereby meeting the demands of a growing world population.

In the prior art, the documents CN105543085A, US10751715B1, US10336978B2, and EP3819367A1 show examples of automated systems of bioreactors where parameters and values of different nature are periodically taken as a basis for adapting the process in the successive steps, where by means of electrodes a current- or potential-based excitation signal is applied to the cell culture based on target vs measured parameters. Further US10751715B1 discloses the measurement of impedance.

### Summary of the invention

It is an object of the present invention to overcome the disadvantages and technical problems known from the prior art. In particular, the aim is to provide an accurate and efficient industrial bioreactor with an automated optimization process allowing to provide a reliable, controllable, and automated detection of optimal processing parameters for an industrial cultivation process in a bioreactor, and thus an optimized steering of the cultivation process in the bioreactor. It is a further object of the invention to provide an automatically controlled and reproducible, nsPEF induced growth stimulation for cell cultures in a bioreactor, in particular for electric fields and PEF applications having dedicated stimulation windows for controllable and reliable growth and/or compound stimulation.

According to the present invention, the objects of the invention can be realized and achieved by means of the elements and combinations particularly depicted in the independent and dependent claims. It is to be understood that both, the foregoing general description and the following detailed description are exemplary and explanatory only and are not restrictive of the invention as described.

According to the present invention, the above-mentioned objects for a method for an industrial bioreactor with a dual cycle-controlled process providing a cultivation process for cell cultures, cell components or metabolic products of the cells in a nutrient medium and appropriate method are achieved, particularly, in that the bioreactor comprises a reactor vessel providing controlled bioreactor conditions for the cultivation process, a control unit connected to sensory devices measuring sensory parameter values comprising at least measures related to the composition of the nutrient medium and/or concentration of the nutrient medium and/or oxygen and/or temperature and/or pH-value and/or sterility, and transmitting them to the control unit, wherein the control unit controls and/or steers the cultivation process by adjusting operational parameters of the bioreactor affecting the measured sensory parameter values, the dual cycle-controlled process comprising the steps of adjusting, by a cultivation optimization cycle optimizing cultivation performance of the cultivation process operational first parameters of the bioreactor identifying a biologically optimized treatment window, and optimizing, by a treatment optimization cycle, a treatment process applied to the cell culture during the cultivation process within the optimized treatment window by applying operational second parameters of the bioreactor, measuring first sensory parameters by first sensory devices, in the cultivation optimization cycle, by capturing the cultivation performance of the cultivation process, wherein the first sensory parameters are transmitted to a fist analyzer reconciling between a target cultivation performance and the measured cultivation performance and if the target cultivation performance is not met, the operational first parameters are adjusted and the measuring and reconciliation is reiterated, measuring bioimpedance by a measuring device of the first sensory devices measuring the cultivation performance of the cultivation process by means of the first sensory parameters by detecting their response to electric excitation, where by means of electrodes a current- or potential-based excitation signal is applied to the cell culture and the response is measured converting the charge to ionic charge and vice versa providing detection of at least cell number and/or cell size and/or cell viability of the cells, and triggering, if the target cultivation performance is met, the treatment optimization cycle, the treatment optimization cycle comprising measuring second sensory parameters by second sensory devices capturing the treatment performance of the treatment process by measuring the treatment induced deviation in the cultivation performance, wherein the second sensory parameters are transmitted to a second analyzer reconciling between a target treatment performance and the measured treatment performance and if the target treatment performance is not met, the operational second parameters are adjusted and the measuring and reconciliation is reiterated, otherwise the dual cycle-controlled optimization process is completed. One of the advantages of the invention is, inter alia, the inventive the industrial bioreactor with a dual cycle-controlled optimization process allows the first time to provide a reliable and controllable detection of optimal processing parameters for an industrial cultivation process in a bioreactor. This is achieved by detecting in a first step the optimal biological treatment window based on the cell characteristics of the cells to be cultivated. In a second step, the technical treatment is optimized, i.e. the operational parameters of the treatment to be applied. This first step can continue during the whole treatment and, thus, can serve to identify the treatment stop while the second step allows to quantify the process efficiency and thus detects the percentage increase of the cultivation/growth efficiency and thus further also allows the prediction of the process runtime.

As an embodiment variant, the control unit can e.g. comprise a machine-learning or artificial-intelligence based module capturing the measured treatment performance measured by means of dielectric spectroscopy system and/or the measured treatment performance measured by means of the flow cytometer, wherein the operational first parameters and/or operational second parameters are automatically adapted by the control unit, wherein at least the first and secondary sensory parameter are applied as input values to the machine-learning or artificial-intelligence based module, and wherein the output of the machine-learning or artificial-intelligence based module triggers the adjustment of the operational first parameters and/or operational second parameters until the target cultivation performance with the applied treatment process is reached. For example, at least some of the measured sensory parameters can serve as input to the machine-learning module, while at least some of operational parameters are subject to the output-values of the machine-learning or artificial-intelligence based module. The machine-learning module can e.g. be based on supervised or unsupervised learning structures. As an example among other suitable machine-learning structures, the machine-learning module can e.g. convert the measured first and/or second sensory parameters into a sequence of assigned binary input codes and process the sequence of the assigned binary input codes by applying a maximum likelihood parameter estimation for the training of a multi-dimensional data structure of the machine-learning module with the variable hidden Markov model parameters, wherein the elements of the sequence of storable parameter states of the Markov chain are assumed to be independent measurements of each other and wherein the model parameters of the multi-dimensional data structure are varied by maximizing the multiplied product of the probabilities in order to obtain the trained model parameters of the multi-dimensional data structure. The model parameters of the multi-dimensional data structure can e.g. be iteratively varied until a predefined convergence threshold is triggered. For determining said threshold value of a score indicating or providing a measure for the optimization of the operational fist and/or second operational parameters, an averaging process can e.g. be applied based on the different pattern of first and/or second sensory parameters of the sensory and/or measuring data of an identified time frames. In an embodiment variant, the sensitivity of the chosen operational parameters can e.g. be automatically tuned based on dynamic adjustments of the threshold value. This embodiment variant has inter alia the advantage, that the convergence speed by training the variable hidden Markov model parameters of the multi-dimensional data structure can be optimized. The embodiment variant has, inter alia, the advantage that it provides a novel method and bioreactor for automated detection, measuring and triggering of an optimized setting for the first and second operational parameters affecting the cultivation of the cell cultures and the applied treatment used in industrial bioreactor. It provides an efficient automated system for controlling and monitoring small- to large-scale industrial bioreactors (e.g. used for cultured meat production), which typically are difficult to handle. In addition, this embodiment variant has, inter alia, the advantage that the AI integration allows to serve finally to treat also completely unknown organisms without evaluating appropriate settings for the first and/or second operational parameters of the bioreactor empirically, since the system optimizes itself by means of the actual/target comparison.

***Brief Description* of *the Drawings***

The present invention will be explained in more detail, by way of example, with reference to the drawings in which:

Figure 1 shows a diagram schematically illustrating a biological membrane. Core for understanding how cells interact with their environment is the boundary that separates the cell, i.e. its membrane. Cell membranes (fig. 1) are composed of a series of polar lipid molecules that have hydrophobic and hydrophilic regions organized into a bilayer. This structure displays self-organizing properties in an aqueous environment that results in a boundary condition separating the cytoplasm from extracellular fluids. A very similar arrangement of amphipathic lipid molecules within the cell serves to separate organelles from the cytoplasm. These membranes are critical for selective transport into and out of the cell. In addition to the phospholipid bilayer, a host of other lipids, proteins and carbohydrates can be identified. These molecules are highly variable in their distribution along the membrane, each serving different functions ranging from signaling molecules to structural support etc.. Transmembrane protein channels are of particular interest to the topic. The cell membrane, as shown in figure 1, is a highly complex structure whose fundamental unit is the phospholipid molecule (bottom right of figure 1). The amphipathic structure of this molecule results in the formation of a bilayer in aqueous solution. Embedded within the membrane, multiple additional lipids, proteins and carbohydrates serve to communicate with the external environment.

Figure 2 shows a diagram schematically illustrating ion channels classified by the stimulus which modulates their activity. Transmembrane proteins, to a large degree, dictate the function of the cell. Some serve as signal conduits, whereby extracellular molecules interact with the protein leading to a specific intracellular effect. Others serve as selectivity filters responsible for modulating, either passively or actively, the passage of ions. These ion channels are highly variable and are critical for many cell processes. Ion channels fluctuate between open and closed states, and these states can be influenced through multiple mechanisms, as illustrated in figure 2. Some examples include voltage-gated channels, mechanically-gated channels, or ligand-gated channels. Upon activation, these channels become permeable to specific ions. They can be highly specific, meaning they allow just one type of ion to pass, or charge-specific allowing either cations or anions to pass. Voltage-gated channels can be activated by fluctuations in a cells transmembrane potential. Ligand-gated channels are activated through signal molecules that bind on extra- or intracellular sites of the channel. Mechanically-gated channels are modulated through changes in shape associated a physical stimulus. The selective nature of these ion channels is responsible for the generation of a transmembrane potential, which is equally present and varies among intracellular organelles as it is across the plasma membrane. The chemical basis of this voltage-gradient, known as the resting membrane potential, is the uneven distribution of ions inside and outside of the cell. The electrical properties of ions, combined with the conductive properties of protein channels and the insulating properties of the membranes lipid bilayer, have allowed the cell to be modeled as an electric circuit. This has been useful when modelling electrical communication between cells for example, but also when developing technical cultivation and/or treatment strategies to modulate cell activity and cell cultivation.

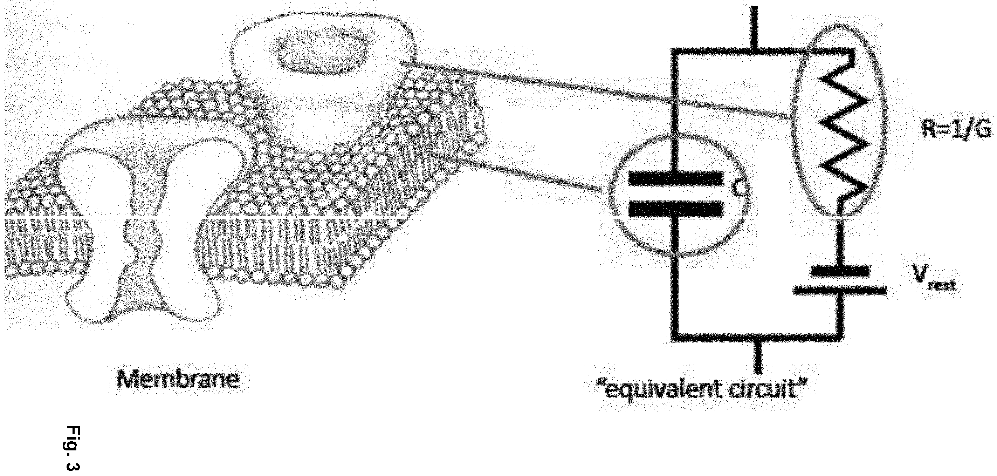

Figure 3 shows a diagram schematically illustrating the cell working as an electrical circuit, captured by the Hodgkin-Huxley parametrization. The Hodgkin-Huxley parametrization (introduced by A. Hodgkin and A. Huxley) proposes a working structure or model to capture the ionic mechanisms responsible for the propagation of electrical impulses along a giant squid axon. As a general overview shown by figure 3, the Hodgkin-Huxley parametrization assumes the lipid bilayer as parallel capacitors capable of storing charge and ion channels as variable resistors capable of passing current. The transmembrane potential is generated across the cell membrane and fluctuates according to the activity of the ion channels. This model assumptions are important for capturing or modelling certain biological phenomena such as electrochemical communication between cells, particularly between electrically excitable cells in nervous and muscular tissues. Another application for this model extends into the technical field interesting for the present application, for modulating and steering cellular activities using applied electric fields in bioreactors. In figure 3, the lipid bilayer is represented as a pair of parallel capacitors and the transmembrane ion channels are represented as variable resistors.

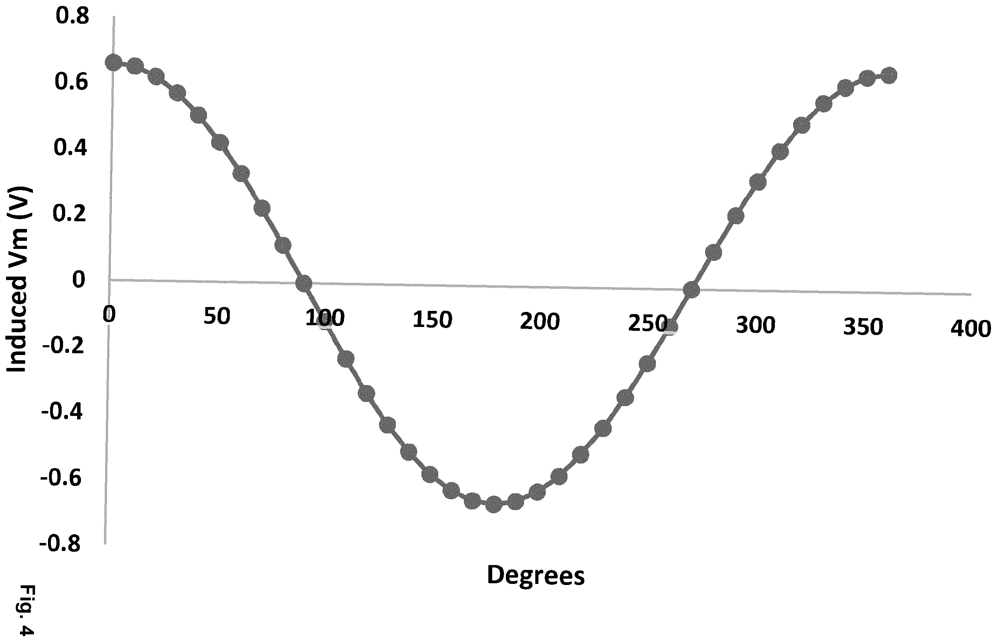

Figure 4 shows a diagram schematically illustrating the electric field interaction with the cell membrane. When cells are exposed to an electric field, an electric force is generated, which acts on ions both inside the cell and in the external media. Because ions are charged molecules, this force causes them to move along the electric field lines. Whereas the extra- and intracellular solutions are conductive, the cells lipid membrane is non-conductive. As a result these ions will accumulate along the membrane and generate a large transmembrane potential. At a certain point, which is assumed in the prior art to be approximately 1V, the induced voltage exceeds the membrane capacitance and breakdown of the membrane occurs. When considering the diameter of the membrane is approximately 10 nm, the electric field strength associated with this threshold is on the order of a MV/cm. This process, which is termed electroporation or electropermeabilization (see above) is associated with enhanced membrane permeability. To capture this effect, the Schwan parametrization respectively relation was developed, given by:

$$V_m = \frac{3}{2} ERcos\theta$$

The above relation is limited to capturing spherical cells, and states that the induced membrane potential (Vm) is proportional to the radius of the cell (R) and the applied electric field (E), and will not be uniform along the cell membrane, as shown in figure 4. Extensions of this model have been developed to allow capturing more complex structures, such as irregular-shaped cells that can't be accurately described as spherical or ellipsoid. The Schwan relation (steady-state) allows capturing the effect of an applied electric field (E) on the transmembrane potential (Vm) given a cell radius of (R) as a function of the angle ($\theta$). In figure 4, the induced membrane potential varies along the cell membrane. This example applies the Schwan parametrization to a spherical cell with a radius of 10 $\mu$m and an applied field intensity of 44 kV/cm. In prior art, there has been a shift in methodology toward capturing or parametrizing the biomolecular events occurring when an electric field interacts with a lipid bilayer. Molecular dynamics (MD) simulations have helped to model some of the events associated with electropermeabilization, and can be used to visualize the dynamics associated with membrane breakdown. Up to now, limitations in the computational power have restricted MD to a period less than 1 millisecond following pulse delivery; however, a multitude of effects have been reported over much longer time periods. These effects appear to be highly dependent on several factors such as; pulse duration, electric field strength, number of pulses, and frequency of pulses delivered.

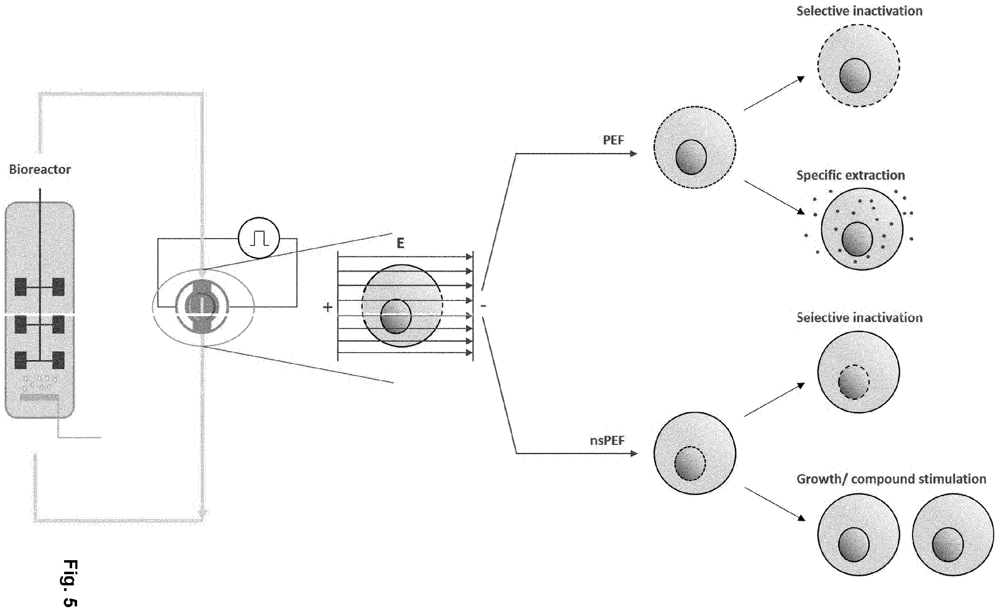

Figure 5 shows a diagram schematically illustrating the exemplary working principles of PEF/nsPEF based processing of cultivated cells and their respective effects.

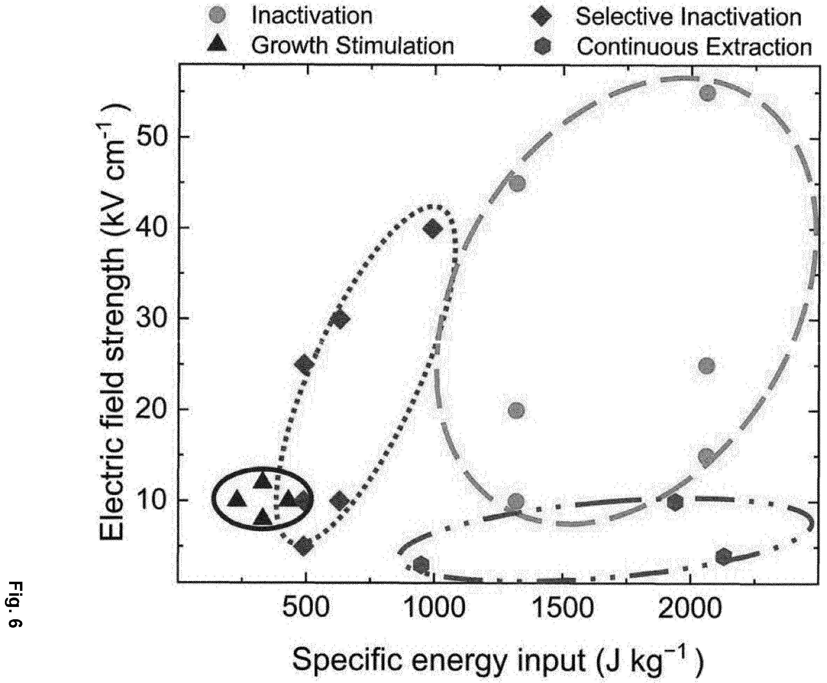

Figure 6 shows a diagram schematically illustrating treatment windows for selective inactivation, inactivation by the example of microbial flora and microalgae Chlorella vulgaris, continuous extraction of high value-added ingredients, and growth stimulation.

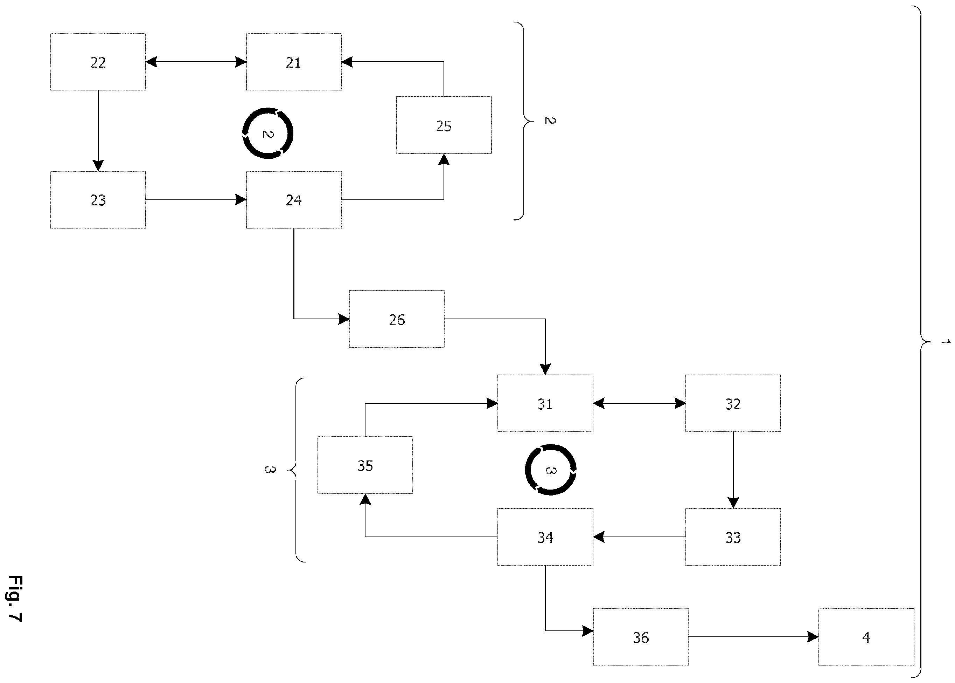

Figure 7 shows a block diagram schematically illustrating an embodiment variant of the inventive industrial bioreactor 1 with a dual cycle-controlled optimization process providing an optimized cultivation process 21 for cell cultures, cell components or metabolic products of the cells in a nutrient medium 11. The dual cycle-controlled optimization process comprises a cultivation optimization cycle 2 optimizing cultivation performance of the cultivation process 21 by adjusting operational first parameters of the bioreactor 1 identifying a biologically optimized window for cultivation and/or treatment, and a treatment optimization cycle 3 optimizing a treatment process 32 applied to the cell culture during the cultivation process 21 within the optimized treatment window by applying operational second parameters of the bioreactor 1, wherein the cultivation optimization cycle 2 comprises measuring 22 first sensory parameters by first sensory devices capturing the cultivation performance of the cultivation process 21, wherein the first sensory parameters are transmitted to a fist analyzer 23 reconciling between a target cultivation performance and the measured cultivation performance and if the target cultivation performance is not met, the operational first parameters are adjusted and the measuring 22 and reconciliation 24 is reiterated. If the target cultivation performance is met, the treatment optimization cycle 3 is triggered comprises measuring 32 second sensory parameters by second sensory devices capturing the treatment performance of the treatment process 31 by measuring the treatment induced deviation in the cultivation performance, wherein the second sensory parameters are transmitted to a second analyzer 33 reconciling between a target treatment performance and the measured treatment performance and if the target treatment performance is not met, the operational second parameters are adjusted and the measuring 32 and reconciliation 34 is reiterated, otherwise the dual cycle-controlled optimization process is completed.

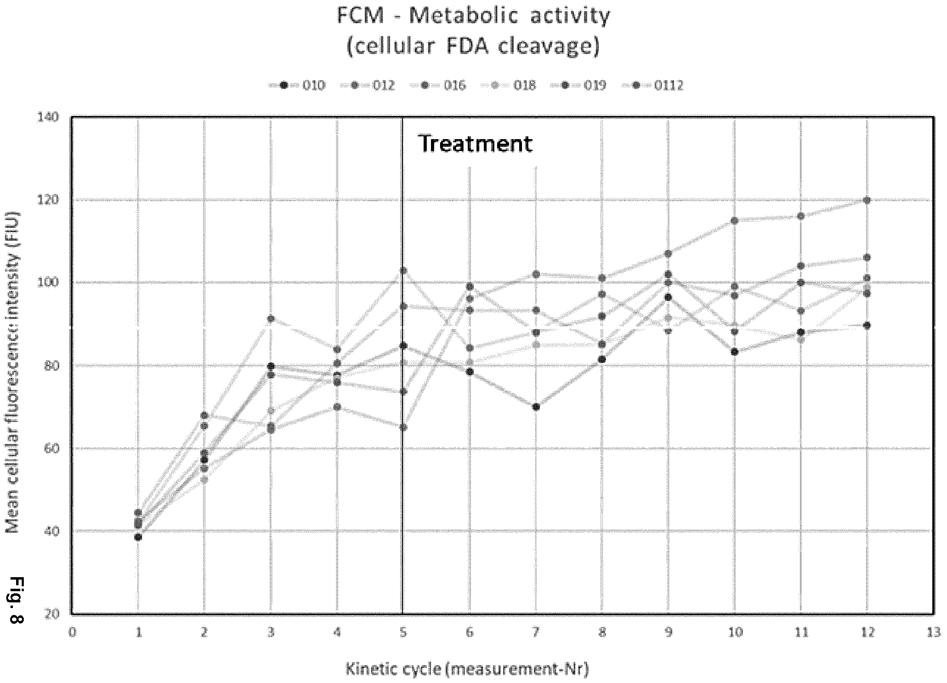

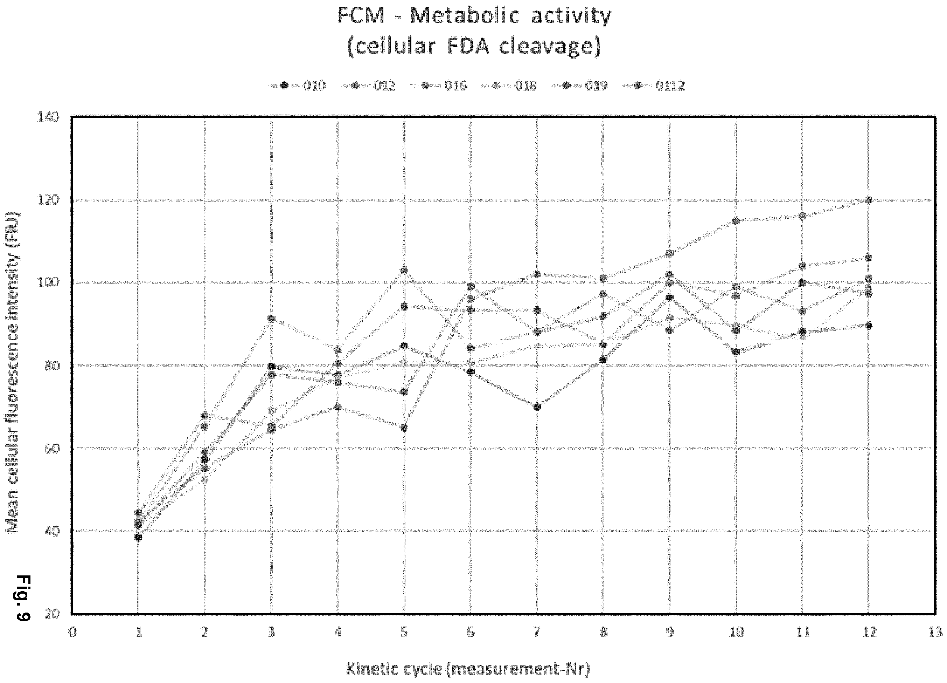

Figures 8 and 9 show a diagram schematically illustrating measuring data of the industrial treatment cycle, where the physical and chemical characteristics of a population of cells is measured by flow cytometry (FC) measurements. The diagram line with the reference number 012 shows correctly treated cell samples, the diagram lines with the reference numbers 018 and 019 show control samples without applied treatment process, and the diagram lines with the reference numbers 010, 016 and 0112 show wrong treated cell samples, i.e. having an applied treatment with not the correct or optimized combination of amplitude, pulse length and number of pulses. In the flow cytometry measurement process, the samples containing the cells are suspended in a fluid and injected into the flow cytometer measuring device. The sample is focused to ideally flow one cell at a time through a laser beam, where the light scattered is characteristic to the cells and their components. Cells are labeled with fluorescent markers so light is absorbed and then emitted in a specific band of wavelengths. In the diagram, the blue vertical line marks the start of the treatment process in the bioreactor.

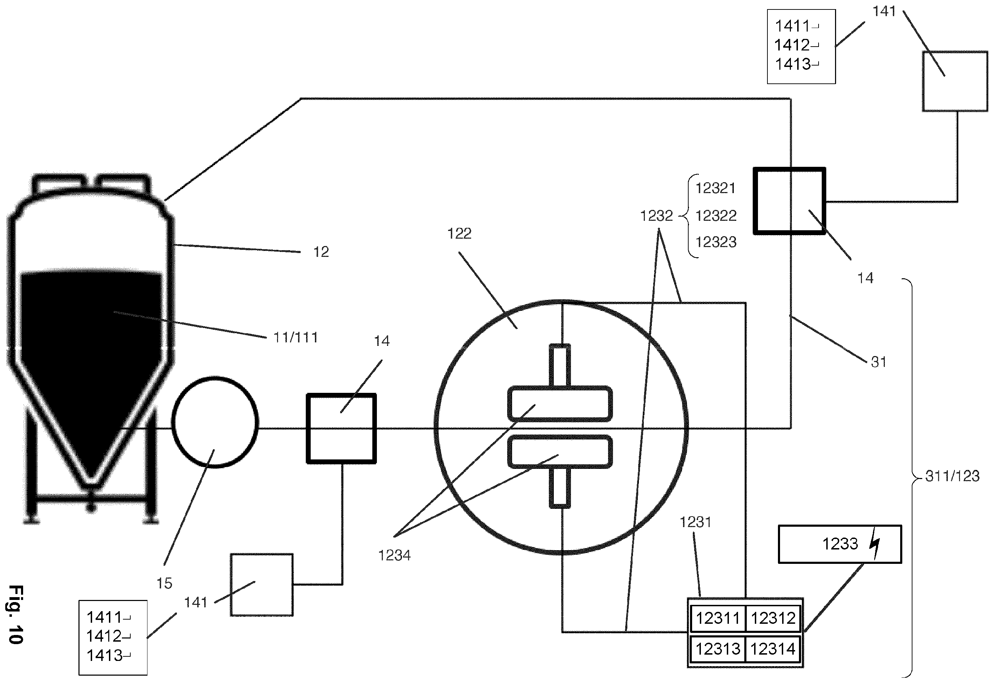

Figure 10 shows a block diagram schematically illustrating an industrial liquid bioreactor 1, which type of bioreactor can be used for the present invention, where the cell cultures or micro-organisms are grown in submerged in liquid medium. The industrial liquid bioreactor 1 comprises devices as a pump 15, sensors or sensory devices 14, treatment device 311, and a reactor vessel 12 containing the liquid nutrient medium 11/111. Such industrial liquid or solid state bioreactors 1 are vessels that are designed to provide an effective environment for enzymes or whole cells/cell structures to transform biochemicals into products. Inactivation of cells or sterilization is one of the applications which can be carried out in the bioreactor such as in water treatment. Such industrial bioreactors can also be used for a great variety of other bioreactor applications, including applications for cell growth, enzyme production, biocatalysis, biosensors, food production, milk processing, extrusion, tissue engineering, algae production, protein synthesis, and anaerobic digestion.

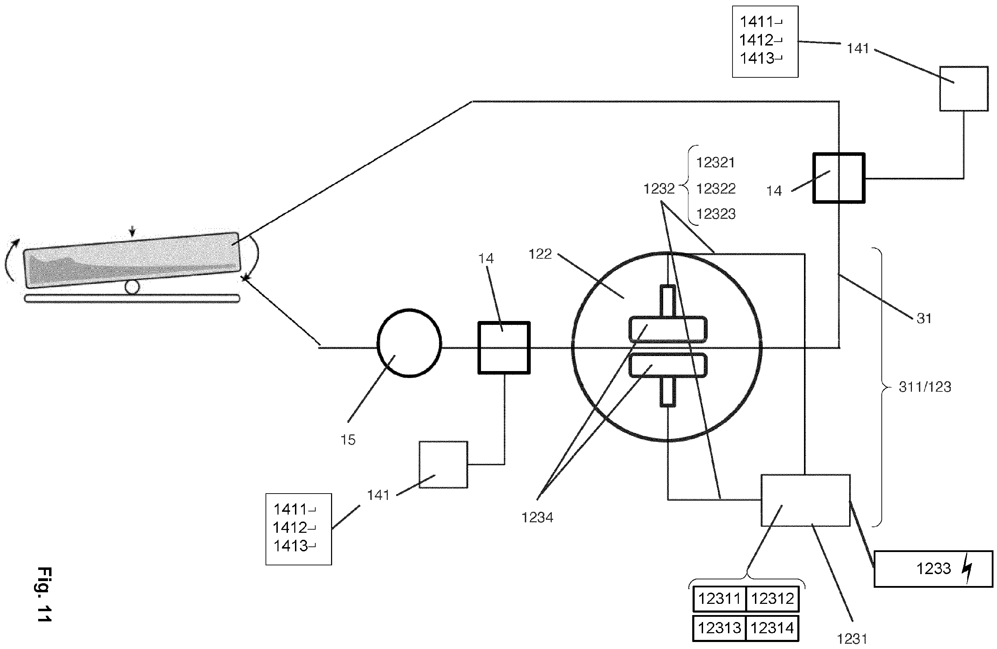

Figure 11 shows a block diagram schematically illustrating an industrial wave bioreactor 1 which type of bioreactor is also usable for the present invention. Liquid bioreactor are not suitable for all cultivation, since industrial cultivation technology for the culture of animal, plant and insect cells is dependent on the batch volume required. Though, devices such as spinner flasks, roller bottles, T-flasks, and similar systems can be used, these devices can typically only produce 1 to 2 L of culture per batch due to their inherently limited oxygen transfer capabilities. For example, spinner flasks, though very popular for suspension culture, as well for anchorage-dependent systems on microcarriers, are typically only useful for volumes less than 1 L. For larger volumes, it is usually necessary to use stirred-tank bioreactors that are modified to reduce shear forces. These bioreactors are complex and expensive devices, yet they often do not provide an ideal environment for cell growth due to high local fluid shear and the use of bubble aeration. For the industrial production of 1 to 100 L of cell culture, which is, for example, a typical volume required for protein characterization, inoculum propagation, and pilot-scale production, the adaption of stirred tank technology to cell culture is a futile exercise because this design has intrinsically high local shear rates making scale-up very difficult. Instead, it is critical to understand the special demands of cell culture to directly satisfy these needs. These demands include shear sensitivity, use of bubble-free aeration and pre-sterilized media, and small oxygen uptake rates. For industrial use it is further essential that any cultivation system is as simple as possible to operate. In this sense, many otherwise useful devices, such as fluidized bed bioreactors and hollowfiber systems, have turned out to be too complex to displace the spinner flask as the workhorse of cell cultivation. However, the use of wave bioreactor 1 in industrial application allows sometimes to overcome these problems. The wave bioreactor typically consists of a presterilized chamber 121, for example an inflatable, flexible plastic chamber, that is partially filled with media and inoculated with cells. The remainder of the chamber 121 is inflated with air. The air is continuously passaged through the headspace during the cultivation. Mixing and mass transfer are achieved by rocking the chamber back and forth. This rocking motion generates waves at the liquid-air interface, greatly enhancing oxygen transfer. The wave motion also promotes bulk mixing, and off-bottom suspension of cells and particles. This concept of using rocking for agitation can e.g. be used for the agitation of liquids in industrial assay plates and gels. The rocking device shown in figure 9 can e.g. consist of a platform that rotates in one axis through an angle that can be adjusted, for example, between 5° to 10°. Pneumatic bellows can be used to rock the platform, for example, at a rocking rate that can be adjusted from 5 to 40 rocks/minute (rpm). If a pneumatic system is used, this allows to ensure that unit does not generate any heat during operation, enabling it to be placed inside an incubator. A typically pre-sterilized disposable cell culture chamber 121 is placed on the platform which is partially filled with media 111 and then inflated using the integral sterile inlet filter. Air can be continuously passaged through the headspace of the chamber 121. This airflow provides oxygenation 141122 and gas exchange for pH control 141121 and $CO_2$ removal. Exhaust air passes through a sterilizing filter and a backpressure control valve. A backpressure control valve can ensure that the chamber 121 is always fully inflated at any airflow. Such a valve can also prevent overinflation and potential bursting of the chamber 121. Temperature 14111 and pH control 141121 can e.g. be achieved by placing the entire unit inside a conventional cell culture $CO_2$ incubator. Alternatively, temperature control 14111 can e.g. be achieved by heating the underside of the culture chamber 121. The wave motion promotes good bulk liquid 111 movement and minimizes the occurrence of temperature gradients. The industrial wave bioreactor 1 further comprises devices as a pump 15, sensors or sensory devices 14, treatment device 311, and as reactor vessel 12 the chamber 121 containing the liquid nutrient medium 11/111.

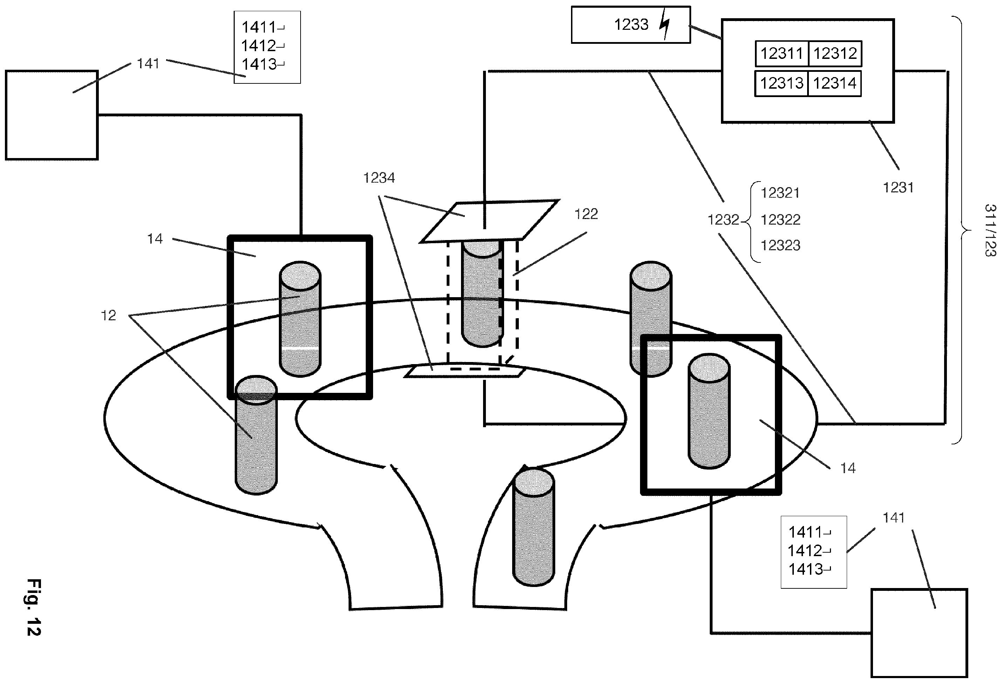

Figure 12 shows a block diagram schematically illustrating a solid-state bioreactor which type of bioreactor is also usable for the present invention. Solid-state bioreactors (SSF: Solid-State Fermentation) have, inter alia, important advantages over the other type of bioreactors with submerged fermentation, such as lower water use and higher volumetric productivity. As a result, SSF technology covers an increasing filed in the industrial application of bioreactors in the past few decades. SSF bioreactors can be based on various types of reactor technology, as for example tray, packed-bed, rotating/stirred-drum, fluidized-bed, rocking-drum, and stirred-aerated bioreactors. Technically important is typically the reactor design, the control of heat and mass transfer, and the applied operational strategies. Both, industrial cultivation of cells as well as the growth of microorganisms can be realized in SSF bioreactors. Important for the industrial applications of SSF bioreactors are, as mentioned, typically the control of the heat transfer on a large scale and to quantitative characterization and measurement of the cell growth. For the latter, one of the technical challenges are e.g. the appropriate selection of nondestructive detection methods during the SSF processes. In general, compared to submerged fermentation, the solid media 112 used in SSF contain less water, however, an important gas phase typically exists between the particles. This feature is of great technical importance because of the poor thermal conductivity of the air compared to the water. Another point is the wide variety of matrices 112 used in SSF bioreactors which vary in terms of composition, size, mechanical resistance, porosity and water holding capacity. All these factors can affect the reactor design and the control strategy for the parameters. Indeed in submerged fermentation, all the media can be considered to be made up essentially of water. Thus, in liquid bioreactors, the temperature 14111 and pH 141121 regulations are typically simple to achieve and pose no problem during the industrial scaling-up of a process. In submerged fermentation, the major difficulty is typically the transfer of oxygen to micro-organisms or cells which depends upon the shape, the size of the reactor and the agitation/aeration system used. This transfer can be measured by a parameter KLa (oxygen transfer coefficient) where its value expresses the capacity of the equipment to transfer oxygen independently of the volume of the reactor and so, constitutes an important parameter used for the scale-up design in submerged fermentation. In contrast to liquid bioreactors, in SSF bioreactors, besides the oxygen transfer 141122 which can be a limiting factor for some designs, the technical problems are more complex affecting the control of two important parameters, namely the temperature 14111 and the water content of the solid medium 112, i.e. the substrate 112. Concerning the various types of solid-state bioreactors, it is important to note that generally many types of reactors are able to run a cultivation process at laboratory-scale with small quantities of medium 112. But the industrial scale-up is often complicated inter alia due to intense heat generation and heterogeneity in the system, which also complicates the technical control of the cultivation process. In industrial solid-state bioreactor, the above-discussed heat and mass transfer problems can, inter alia, attribute to poor aeration. This problem can e.g. be addressed by (i) circulating the air around the substrate layer 112 or (ii) leading the air through the substrate layer 112. For the latter, three possibilities are used in the prior art, which are unmixed, intermittently or continuously mixed beds. However, as already mentioned, the present invention can also be used with solid-state bioreactors. As shown in figure 12, a suitable industrial solid-state bioreactor 1 can e.g. be realized comprising a pump 15, sensors or sensory devices 14, treatment device 311, and as reactor vessel 12 the cultivation containers 121 containing the substrate 11/112.

### Detailed Description of the Invention

Figure 7 illustrate, schematically, an architecture for a possible implementation of an embodiment of the inventive bioreactor 1 with a dual cycle-controlled optimization process providing an optimized cultivation process 21 for cell cultures, cell components or metabolic products of the cells in a nutrient medium 11. The industrial bioreactor 1 comprises a reactor vessel 12 providing controlled bioreactor conditions for the cultivation process 21. The bioreactor is an industrial bioreactor for the production of cells or sell cultures, such as cultured meat production. The bioreactor 1 can e.g. also be realized as a fermenter and/or a germination box in case of seed germination applications. Further, the bioreactor can also be an industrial bioreactor for the production of metabolic products, such as biologically active secondary metabolites (antibiotics, bacterial toxins, immune drugs, and alkaloids), single cell proteins, enzymes, industrial chemicals, biofuel, food, phenolics, feed, and pharmaceutical products. However, it is to be noted, that the present invention can be applied to any kind of liquid bioreactors or solid-state bioreactor, such as Solid-State Fermentation (SSF) bioreactors or Submerged Fermentation (SmF) bioreactors, inter alia, used in the field of waste management technology, and applications including bioremediation, detoxification, bioleaching and biopulping etc.. In a preferred embodiment variant, the bioreactor is used in food production such as cultured meat or fermented food, e.g. tempeh, miso, koji, and soy sauce etc. The bioreactor 1 can e.g. comprise one or a plurality of substrate container 121 (also referred as trays) enterable into the reactor vessel 12, wherein the cell culture is cultivated on a nutrient medium 11 also contained in the substrate container 121. However, the bioreactor can not only be realized as a tray bioreactor, but also e.g. as a packed bed bioreactor, an air pressure pulsation bioreactor, or an intermittent or continuously mixed SSF bioreactors etc. If the bioreactor is used for cultured meat production may comprise a scaffold holding the one or more substrate container 121 comprising a population of cells. The cultured meat product produced in the bioreactor can e.g. have a thickness from about 100 $\mu$m to about 500 mm, or any other size. In an embodiment, a method of producing such a cultured meat product may comprise preparing the scaffold, placing the scaffold into a bioreactor, adding the substrate container 121 with the nutrient medium 11 and the cell culture or population of cells to the bioreactor 1, culturing and cultivating, respectively, the population of cells in the bioreactor containing the scaffold for a period of time, hereby forming the cultured meat product, and removing the cultured meat product from the bioreactor 1. During the cultivation process 21 in the bioreactor 1, a treatment 31 can be applied to the cell cultures, as described below. The cultured meat product can e.g. be configured to mimic the taste, texture, size, shape, and/or topography of a traditional slaughtered meat. As used herein, the term "traditional slaughtered meat" means one or more types of meat obtained from a once-living animal for the purpose of consumption. Such meat is generally, although not always, obtained from livestock, fish, or other animals raised or slaughtered primarily for food production purposes. Non-limiting examples of traditional slaughtered meat include chicken, turkey, pork, steak, fish, and the like. Traditional slaughtered meat is generally appropriate for consumption by one or more mammal species. Further as used herein, the term "cultured meat product" means a meat product that is produced by human or machine intervention, rather than grown as a natural component of a living animal. A cultured meat product is thus not obtained directly from the slaughter of a living animal, but rather by an artificial cultivation process e.g. induced in a controlled bioreactor environment. Like traditional slaughtered meat, a cultured meat product is generally appropriate for consumption by one or more mammal species.

A control unit of the bioreactor 1 is connected to sensory devices 14 measuring sensory parameter 141 values comprising at least measures related to the composition 1411 of the nutrient medium 11 and/or concentration 1412 of the nutrient medium 11 and/or oxygen 1413 and/or temperature 1414 and/or pH-value 1415 and/or sterility 1416 and transmitting them to the control unit. The cultivation process 21 is controlled and/or steered by the control unit by adjusting operational parameters of the bioreactor 1 affecting the measured sensory parameter 141 values.

The dual cycle-controlled optimization process comprises a cultivation optimization cycle 2 optimizing cultivation performance of the cultivation process 21 by adjusting operational first parameters of the bioreactor 1 identifying a biologically optimized window for cultivation and/or treatment. Further, the dual cycle-controlled optimization process comprises a treatment optimization cycle 3 optimizing a treatment process 32 applied to the cell culture during the cultivation process 21 within the optimized treatment window by applying operational second parameters of the bioreactor 1.

The cultivation optimization cycle 2 comprises measuring 22 first sensory parameters by first sensory devices of the sensory devices 14 capturing the cultivation performance of the cultivation process 21. The first sensory parameters are transmitted to a fist analyzer 23 reconciling between a target cultivation performance and the measured cultivation performance and if the target cultivation performance is not met, the operational first parameters are adjusted and the measuring 22 and reconciliation 24 is reiterated. The first sensory devices measuring the cultivation performance of the cultivation process 21 by means of the first sensory parameters can e.g. comprise a measuring device for bioimpedance measurement detecting their response to electric excitation, where by means of electrodes a current- or potential-based excitation signal is applied to the cell culture and the response is measured converting the charge to ionic charge and vice versa providing detection of at least cell number and/or cell size and/or cell viability of the cells.

If the target cultivation performance is met, the treatment optimization cycle 3 is triggered comprises measuring 32 second sensory parameters by second sensory devices of the sensory devices 14 capturing the treatment performance of the treatment process 31 by measuring the treatment induced deviation in the cultivation performance. The second sensory parameters are transmitted to a second analyzer 33 reconciling between a target treatment performance and the measured treatment performance and if the target treatment performance is not met, the operational second parameters are adjusted and the measuring 32 and reconciliation 34 is reiterated, otherwise the dual cycle-controlled optimization process is completed.

For example, the treatment process 31 can e.g. comprise applying nanosecond pulsed electric fields (nsPEF) to the cell culture using at least two applied electrodes, the electric fields being applied by coupling one electrode to higher voltage and one electrode to ground or lower voltage, and the pulsed electric fields having a definable shape and/or frequency and/or strength. As a variant, the control unit can e.g. comprise predefined basic nanosecond pulsed electric fields settings for each possible cell type.

The nanosecond pulsed electric fields (nsPEF) can e.g. be applied by electrodes to the bioreactor 1, as such. If the bioreactor comprises one or more substrate container 121 holding the nutrient medium 11 and the cell culture, the electrodes can also be applied to each substrate container 121, e.g. by integrating them into possible walls of the substrate container 121. Finally, the bioreactor 1 can also include a pulsed electric field (PEF) station or be included in a pulsed electric field (PEF) station providing the required pulsed electric fields. In the latter cases, the biological materials can e.g. flow into the PEF station via an inlet, may be treated, and may then be released via an outlet. The PEF generation can e.g. be provided by a pulse generator, where the PEF treatment is effective in a particular treatment zone comprising the cell culture. In particular, materials contained in or passing through the treatment zone are subjected to non-arcing electric field pulses generated by the pulse generator. The electric field pulses can e.g. be generated by applying a voltage pulse to the electrodes, the pulse can e.g. have a square-wave shape. However, the pulses may also have an exponentially decaying or oscillatory shape. Further, the pulses may be monopolar, bipolar, or even instant reverse charges. The electric field pulses can e.g. be of a preferred duration of 10 to several 100 nanoseconds, however dependent on the treatment, the pulse duration can e.g. lie also in the range of 2 to 15 microseconds with a peak field strength of 15 to 100 kV /cm etc.. The resulting duration of treatment can e.g. be a function of the shape of the treatment zone (e.g., electrodes) and the characteristics of the electric field pulses. Preferrable, the treatment is applied during a larger part or the whole cultivation process 21. The pulse generator can e.g. be coupled to a power supply, which the pulse generator uses to generate a series of high voltage non-arcing electric field pulses across electrodes associated with the treatment zone. Depending on the power supply used, a voltage transformer can e.g. be included, coupled between the power supply and the pulse generator. The pulse generator can include a bank of capacitors and switching circuitry that may connect the bank of capacitors across the electrodes to create the pulses within the treatment chamber. A switching circuitry can e.g. be controlled by a controller that has as an input a signal from a signal generator. By varying the characteristics of the signal from the signal generator, the characteristics of the pulses in the treatment zone can be varied.

As an embodiment variant, in the bioreactor vessel 12 or the substrate container 121 can be disposed electrodes, one of the electrodes coupled to a higher voltage and the other the electrodes coupled to ground or a lower voltage. Insulators can be disposed at either side of the electrodes and between the electrodes. The insulators, as well as the substrate container 121, which can e.g. be made of an insulating material, isolate the electrodes from couplings which may be attached or secured to either end of the substrate container 121. Similarly, the insulator and the substrate container 121 space the electrodes to define the treatment zone disposed therebetween. In operation, the biological materials, i.e. the cell culture, are to be treated in the treatment zone e.g. during the cultivation process 21.

The treatment performance can e.g. be measured by means of dielectric spectroscopy system measuring the dielectric properties of the cells as a function of frequency, wherein the frequency-dependent permittivity in a target range is measured, and wherein the measured amplitude or signal intensity serving as a measured target parameter value for the performance of the treatment. The target range can e.g. lie at 0.1-30 MHz. Thus, the treatment performance can e.g. measured by means of a flow cytometer as at least one of the second sensory devices measuring metabolic activity based on a fluorescence assay, a conversion of the fluorescent dye and the signal intensity serving as a measured target parameter value for the performance of the treatment. The fluorescence assay can e.g. be fluorescein diacetate (FDA). The flow cytometer can e.g. comprise at least a measuring system and a detector and an amplification unit, the flow cytometer being connected to and transferring measuring signals to the control unit for analysis of the transmitted signals. The measuring system measures impedance or conductivity can e.g. use optical systems realized at the bioreactor 1, the optical systems emitting light signals. The detector can e.g. comprise an analog-to-digital conversion ADC system converts analog measurements of forward-scattered light (FSC), side-scattered light (SSC), and dye-specific fluorescence signals into digital signals being processable by the control unit. The amplification unit can e.g. be linear or logarithmic realized.

Thus, the industrial bioreactor with a dual cycle-controlled optimization process provides a reliable and controllable detection of optimal processing parameters for an industrial cultivation process in a bioreactor by detecting in a first step the optimal biological cultivation window based on the cell characteristics of the cells to be cultivated. In a second step, the technical treatment is optimized, i.e. the optimal treatment window for the operational parameters of the treatment to be applied, is automatically detected. This first step can continue during the whole treatment and, thus, can serve to identify the treatment stop while the second step allows to quantify the process efficiency and thus detects the percentage increase of the cultivation/growth efficiency and thus further also allows the prediction of the process runtime. Therefore, a phase angle of the cell culture can e.g. be measured by the bioimpedance measurement 22, wherein the phase angle is correlated with the cell viability, and wherein with increasing measured phase angle the treatment 31 is applied while with decreasing measured phase angle, the treatment 31 is stopped. The treatment performance can e.g. be detected or measured to be optimized, if an acceleration of the cultivation process 21 and/or a targeted biological growth is measured in the bioreactor 1 based on the measured second sensory parameter values.

Finally, as a further suitable embodiment4 variant, the control unit can e.g. comprise a machine-learning or artificial-intelligence based unit capturing the measured treatment performance measured by means of dielectric spectroscopy system and/or the measured treatment performance measured by means of the flow cytometer. In this embodiment variant, the operational first parameters and/or operational second parameters are automatically adapted by the control unit, wherein at least the first and secondary sensory parameter are applied as input values to the machine-learning or artificial-intelligence based unit, and wherein the output of the machine-learning or artificial-intelligence based unit triggers the adjustment of the operational first parameters and/or operational second parameters until the target cultivation performance with the applied treatment process 32 is reached. As already discussed above, the machine-learning module can e.g. be based on supervised or unsupervised learning structures. As an example, machine-learning module can e.g. convert the measured first and/or second sensory parameters into a sequence of assigned binary input codes and process the sequence of the assigned binary input codes by applying a maximum likelihood parameter estimation for the training of a multi-dimensional data structure of the machine-learning module with the variable hidden Markov model parameters, wherein the elements of the sequence of storable parameter states of the Markov chain are assumed to be independent measurements of each other and wherein the model parameters of the multi-dimensional data structure are varied by maximizing the multiplied product of the probabilities in order to obtain the trained model parameters of the multi-dimensional data structure. The model parameters of the multi-dimensional data structure can e.g. be iteratively varied until a predefined convergence threshold is triggered. For determining said threshold value of a score indicating or providing a measure for the optimization of the operational fist and/or second operational parameters, an averaging process can e.g. be applied based on the different pattern of first and/or second sensory parameters of the sensory and/or measuring data of an identified time frames. In an embodiment variant, the sensitivity of the chosen operational parameters can e.g. be automatically tuned based on dynamic adjustments of the threshold value. This embodiment variant has inter alia the advantage, that the convergence speed by training the variable hidden Markov model parameters of the multi-dimensional data structure can be optimized. The embodiment variant has, inter alia, the advantage that it provides a novel method and bioreactor for automated detection, measuring and triggering of an optimized setting for the first and second operational parameters affecting the cultivation of the cell cultures and the applied treatment used in industrial bioreactor. It provides an efficient automated system for controlling and monitoring small- to large-scale industrial bioreactors (e.g. used for cultured meat production), which typically are difficult to handle. In addition, this embodiment variant has, inter alia, the advantage that the AI integration allows to serve finally to treat also completely unknown organisms without evaluating appropriate settings for the first and/or second operational parameters of the bioreactor empirically, since the system optimizes itself by means of the actual/target comparison.

**List of References**

1 Industrial bioreactor

    11 Nutrient Medium

        111 Liquid medium
        112 Substrate

    12 Reactor vessel

        121 Substrate Container
        122 Treatment Zone / Treatment Chamber
        123 Pulsed Electric Field Unit

            1231 Pulse Generator

                12311 Signal Generator
                12312 Controller
                12313 Capacitors
                12314 Switching circuit

            1232 Electric Pulse

                12321 Electric Pulse Shape
                12322 Electric Pulse Frequency
                12323 Electric Field Strength

            1233 Power Supply
            1234 Electrodes

    14 Sensory devices / Sensors
    141 Sensory parameters

        1411 Physical sensory parameters
        14111 Temperature
        1412 Chemical sensory parameters

            141121 pH-Value
            141122 Oxygen
            141123 Composition of the nutrient medium
            141124 Concentration of the nutrient medium

        1413 Biological sensory parameters

            141131 Cell concentration
            1451132 Sterility (e.g. decimal reduction time D-value)

    15 Pump

2 Cultivation Optimization Cycle

    21 Cultivation Process
    22 Measuring of First Sensory Data

        First Sensory Devices
        Bioimpedance measurement device First Sensory Parameters
        Cell Number
        Cell Size
        Cell Viability
        Phase angle

    23 Analyzer
    24 Cultivation Performance Target/Actual Reconciliation

        Actual Cultivation Performance
        Target Cultivation Performance

    25 Threshold/Trigger Not Reached
    26 Threshold/Trigger Reached

3 Treatment Optimization Cycle

    31 Treatment Process

        311 Treatment device

            Bio-based PEF Treatment Device Electrodes
            Pulsed Electric Field (PEF)

                Pulse shape
                Pulse frequency
                Field strength

32 Measuring of Second Sensory Data

    Second sensory devices

        Dielectric spectroscopy system
        Flow cytometer

    Second Sensory Parameters

33 Analyzer
34 Second Target/Actual Reconciliation (target/performance analysis)
35 Threshold/Trigger Not Reached
36 Threshold/Trigger Reached

4 Stop Process

**Claims**

1. Method for an industrial bioreactor (1) with a dual cycle-controlled process providing a cultivation process (21) for cell cultures, cell components or metabolic products of the cells in a nutrient medium (11), the bioreactor comprising a reactor vessel (12) providing controlled bioreactor conditions for the cultivation process (21), and a control unit connected to sensory devices (14) measuring sensory parameter (141) values comprising at least measures related to the composition (1411) of the nutrient medium (11) and/or concentration (1412) of the nutrient medium (11) and/or oxygen (1413) and/or temperature (1414) and/or pH-value (1415) and/or sterility (1416), and transmitting them to the control unit, wherein the control unit controls and/or steers the cultivation process (21) by adjusting operational parameters of the bioreactor (1) affecting the measured sensory parameter (141) values, the dual cycle-controlled process **characterized by** the steps of

    adjusting, by a cultivation optimization cycle (2) optimizing cultivation performance (241) of the cultivation process (21), operational first parameters of the bioreactor (1) by identifying a biologically optimized window for cultivation and/or treatment, and optimizing, by a treatment optimization cycle (3), a treatment process (32) applied to the cell culture during the cultivation process (21) within the optimized treatment window by applying operational second parameters of the bioreactor (1),
    measuring (22) first sensory parameters by first sensory devices of the sensory devices (14), in the cultivation optimization cycle (2) (from before), by capturing the cultivation performance of the cultivation process (21), wherein the first sensory parameters are transmitted to a fist analyzer (23) reconciling between a target cultivation performance and the measured cultivation performance and if the target cultivation performance is not met, the operational first parameters are adjusted and the measuring (22) and reconciliation (24) is reiterated, measuring bioimpedance by a measuring device of the first sensory devices measuring the cultivation performance of the cultivation process (21) by means of the first sensory parameters by detecting their response to electric excitation, where by means of electrodes a current- or potential-based excitation signal is applied to the cell culture and the response is measured converting the charge to ionic charge and vice versa providing detection of at least cell number and/or cell size and/or cell viability of the cells, and

triggering, if the target cultivation performance is met, the treatment optimization cycle (3), the treatment optimization cycle (3) comprising measuring (32) second sensory parameters by second sensory devices of the sensory devices (14) capturing the treatment performance of the treatment process (31) by measuring the treatment induced deviation in the cultivation performance, wherein the second sensory parameters are transmitted to a second analyzer (33) reconciling between a target treatment performance and the measured treatment performance and if the target treatment performance is not met, the operational second parameters are adjusted and the measuring (32) and reconciliation (34) is reiterated, otherwise the dual cycle-controlled optimization process is completed.

2. The method for an industrial bioreactor (1) with a dual cycle-controlled process according to claim 1, **characterized in that** a phase angle of the cell culture is measured by the bioimpedance measurement (22), the phase angle being correlated with a cell viability, wherein with increasing measured phase angle the treatment (31) is applied while with decreasing measured phase angle, the treatment (31) is stopped.

3. The method for an industrial bioreactor (1) with a dual cycle-controlled process according to one of the claims 1 or 2, **characterized in that** the treatment process (31) comprises applying nanosecond pulsed electric fields (nsPEF) to the cell culture using at least two applied electrodes, the electric fields being applied by coupling one electrode to higher voltage and one electrode to ground or lower voltage, and the pulsed electric fields having a definable shape and/or frequency and/or strength.

4. The method for an industrial bioreactor (1) with a dual cycle-controlled process according to claim 3, **characterized in that** the control unit comprises predefined basic nanosecond pulsed electric fields settings for each possible cell type.

5. The method for an industrial bioreactor (1) with a dual cycle-controlled process according to one of the claims 1 to 4, **characterized in that** the treatment performance is measured by means of dielectric spectroscopy system measuring dielectric properties of the cells as a function of frequency, wherein the frequency-dependent permittivity in a target range is measured, and wherein the measured amplitude or signal intensity serving as a measured target parameter value for the performance of the treatment.

6. The method for an industrial bioreactor (1) with a dual cycle-controlled process according to claim 5, **characterized in that** the target range having 0.1-30 MHz.

7. The method for an industrial bioreactor (1) with a dual cycle-controlled process according to one of the claims 3 to 6, **characterized in that** the treatment performance is measured by means of a flow cytometer as at least one of the second sensory devices measuring metabolic activity based on a fluorescence assay, a conversion of a fluorescent dye and a signal intensity serving as a measured target parameter value for the performance of the treatment.

8. The method for an industrial bioreactor (1) with a dual cycle-controlled process according to claim 7, **characterized in that** the fluorescence assay is fluorescein diacetate (FDA).

9. The method for an industrial bioreactor (1) with a dual cycle-controlled process according to one of the claims 7 or 8, **characterized in that** the flow cytometer comprises at least a measuring system and a detector and an amplification unit, the flow cytometer being connected to and transferring measuring signals to the control unit for analysis of the transmitted signals.

10. The method for an industrial bioreactor (1) with a dual cycle-controlled process according to claim 9, **characterized in that** the measuring system measures impedance or conductivity using optical systems emitting light signals.

11. The method for an industrial bioreactor (1) with a dual cycle-controlled process according to one of the claims 9 or 10, **characterized in that** the detector comprises an analog-to-digital conversion (ADC) system converts analog measurements of forward-scattered light (FSC), side-scattered light (SSC), and dye-specific fluorescence signals into digital signals being processable by the control unit.

12. The method for an industrial bioreactor (1) with a dual cycle-controlled process according to one of the claims 9 to 11, **characterized in that** the amplification unit is linear or logarithmic realized.

13. The method for an industrial bioreactor (1) with a dual cycle-controlled process according to one of the claims 1 to 12, **characterized in that** the treatment performance is optimized if an acceleration of the cultivation process (21) and/or a targeted biological growth is measured in the bioreactor (1) based on the measured second sensory parameter values.

14. The method for an industrial bioreactor (1) with a dual cycle-controlled process according to one of the claims 1 to 13, **characterized in that** the bioreactor (1) is realized as a fermenter and/or a germination box in case of seed germination applications.

15. The method for an industrial bioreactor (1) with a dual cycle-controlled process according to one of the claims 1 to 14, **characterized in that** the control unit comprises a machine-learning or artificial-intelligence based unit capturing the measured treatment performance measured by means of dielectric spectroscopy system and/or the measured treatment performance measured by means of the flow cytometer, wherein the operational first parameters and/or operational second parameters are automatically adapted by the control unit (13), wherein at least the first and secondary sensory parameter are applied as input values to the machine-learning or artificial-intelligence based unit, and wherein the output of the machine-learning or artificial-intelligence based unit triggers the adjustment of the operational first parameters and/or operational second parameters until the target cultivation performance with the applied treatment process (32) is reached.

**Patentansprüche**

1. Verfahren für einen industriellen Bioreaktor (1) mit einem doppelten, zyklisch gesteuerten Prozess, der einen Kultivierungsprozess (21) für Zellkulturen, Zellkomponenten oder für Stoffwechselprodukte der Zellen in einem Nährmedium (11) bereitstellt, wobei der Bioreaktor einen Reaktorbehälter (12), der kontrollierte Bioreaktorbedingungen für den Kultivierungsprozess (21) bereitstellt, und eine Steuerungseinheit umfasst, die mit Sensorvorrichtungen (14) verbunden ist, welche Werte sensorischer Parameter (141) messen, die mindestens Messwerte bezüglich der Zusammensetzung (1411) des Nährmediums (11) und/oder der Konzentration (1412) des Nährmediums (11) und/oder des Sauerstoffs (1413) und/oder der Temperatur (1414) und/oder des pH-Wertes (1415) und/oder der Sterilität (1416) umfassen, und diese an die Steuerungseinheit übertragen, wobei die Steuerungseinheit den Kultivierungsprozess (21) durch Einstellen von Betriebsparametern des Bioreaktors (1), welche die gemessenen Werte der sensorischen Parameter (141) beeinflussen, steuert und/oder lenkt, wobei der doppelte, zyklisch gesteuerte Prozess **gekennzeichnet ist durch** die Schritte:

des Einstellens, mittels eines Kultivierungsoptimierungszyklus (2), welcher die Kultivierungsleistung (241) des Kultivierungsprozesses (21) optimiert, der ersten Betriebsparameter des Bioreaktors (1) durch Identifizierung eines biologisch optimierten Fensters für die Kultivierung und/oder Behandlung, und die Optimierung eines Behandlungsprozesses (32) durch einen Behandlungsoptimierungszyklus (3), welcher während des Kultivierungsprozesses (21) auf die Zellkultur angewendet wird, innerhalb des optimierten Behandlungsfensters durch Anwendung der zweiten Betriebsparameter des Bioreaktors (1),

des Messens (22) von ersten sensorischen Parametern durch erste Sensorvorrichtungen der Sensorvorrichtungen (14) in dem (vorherigen) Kultivierungsoptimierungszyklus (2) durch Erfassen der Kultivierungsleistung des Kultivierungsprozesses (21), wobei die ersten sensorischen Parameter an eine erste Analysevorrichtung (23) übertragen werden, die einen Abgleich zwischen einer angestrebten Kultivierungsleistung und der gemessenen Kultivierungsleistung vornimmt, und falls die angestrebte Kultivierungsleistung nicht erreicht wird, die ersten Betriebsparameter angepasst werden und das Messen (22) und Abgleichen (24) wiederholt wird,

des Messens der Bioimpedanz durch eine Messvorrichtung der ersten Sensorvorrichtungen, welche die Kultivierungsleistung des Kultivierungsprozesses (21) mittels der ersten sensorischen Parameter messen, indem deren Reaktion auf elektrische Anregung erfasst wird, wobei mittels Elektroden ein Strom- oder potentialbasiertes Anregungssignal an die Zellkultur angelegt und die Reaktion gemessen wird, indem die Ladung in Ionenladung umgewandelt wird und umgekehrt, was die Erfassung von mindestens der Zellzahl und/oder Zellgröße und/oder Zellviabilität der Zellen bereitstellt, und des Auslösens des Behandlungsoptimierungszyklus (3), falls die angestrebte Kultivierungsleistung erreicht wird, wobei der Behandlungsoptimierungszyklus (3) das Messen (32) zweiter sensorischer Parameter durch zweite Sensorvorrichtungen der Sensorvorrichtungen (14) umfasst, welche die Behandlungsleistung des Behandlungsprozesses (31) erfassen, indem die durch die Behandlung induzierte Abweichung in der Kultivierungsleistung gemessen wird, wobei die zweiten sensorischen Parameter an eine zweite Analysevorrichtung (33) übertragen werden, die einen Abgleich zwischen einer angestrebten Behandlungsleistung und der gemessenen Behandlungsleistung vornimmt, und falls die angestrebte Behandlungsleistung nicht erreicht wird, die zweiten Betriebsparameter angepasst werden und das Messen (32) und Abgleichen (34) wiederholt wird, wobei andernfalls der doppelte, zyklisch gesteuerte Optimierungsprozess abgeschlossen wird.

2. Verfahren für einen industriellen Bioreaktor (1) mit einem doppelten, zyklisch gesteuerten Prozess nach Anspruch 1, **dadurch gekennzeichnet, dass** ein Phasenwinkel der Zellkultur mittels der Bioimpedanzmessung (22) gemessen wird, der Phasenwinkel mit einer Zellviabilität korreliert ist, wobei bei zunehmendem gemessenen Phasenwinkel die Behandlung (31) angewendet wird, während bei abnehmendem gemessenen Phasenwinkel die Behandlung (31) gestoppt wird.

3. Verfahren für einen industriellen Bioreaktor (1) mit einem doppelten, zyklisch gesteuerten Prozess nach einem der Ansprüche 1 oder 2, **dadurch gekennzeichnet, dass** der Behandlungsprozess (31) das Anlegen von Nanosekunden-gepulsten elektrischen Feldern (nsPEF) an die Zellkultur unter Verwendung von mindestens zwei angelegten Elektroden umfasst, die elektrischen Felder durch das Koppeln von einer Elektrode an eine höhere Spannung und von einer Elektrode an Masse oder eine niedrigere Spannung angelegt werden und die gepulsten elektrischen Felder eine definierbare Form und/oder Frequenz und/oder Stärke aufweisen.

4. Verfahren für einen industriellen Bioreaktor (1) mit einem doppelten, zyklisch gesteuerten Prozess nach Anspruch 3, **dadurch gekennzeichnet, dass** die Steuerungseinheit vordefinierte Grundeinstellungen für Nanosekunden-gepulste elektrische Felder für jeden möglichen Zelltyp umfasst.

5. Verfahren für einen industriellen Bioreaktor (1) mit einem doppelten, zyklisch gesteuerten Prozess nach einem der Ansprüche 1 bis 4, **dadurch gekennzeichnet, dass** die Behandlungsleistung mittels eines dielektrischen Spektroskopiesystems gemessen wird, das die dielektrischen Eigenschaften der Zellen als Funktion von der Frequenz misst, wobei die frequenzabhängige dielektrische Leitfähigkeit in einem Zielbereich gemessen wird und wobei die gemessene Amplitude oder die Signalintensität als ein gemessener Zielparameterwert für die Leistung der Behandlung dient.

6. Verfahren für einen industriellen Bioreaktor (1) mit einem doppelten, zyklisch gesteuerten Prozess nach Anspruch 5, **dadurch gekennzeichnet, dass** der Zielbereich 0,1 bis 30 MHz umfasst.

7. Verfahren für einen industriellen Bioreaktor (1) mit einem doppelten, zyklisch gesteuerten Prozess nach einem der Ansprüche 3 bis 6, **dadurch gekennzeichnet, dass** die Behandlungsleistung mittels eines Durchflusszytometers gemessen wird, wobei mindestens eine von einer der zweiten Sensorvorrichtungen gemessene Stoffwechselaktivität auf der Grundlage eines Fluoreszenztests, einer Umwandlung eines Fluoreszenzfarbstoffs und einer Signalintensität als ein gemessener Zielparameterwert für die Leistung der Behandlung dient.

8. Verfahren für einen industriellen Bioreaktor (1) mit einem doppelten, zyklisch gesteuerten Prozess nach Anspruch 7, **dadurch gekennzeichnet, dass** der Fluoreszenztest mit Fluoresceindiacetat (FDA) durchgeführt wird.

9. Verfahren für einen industriellen Bioreaktor (1) mit einem doppelten, zyklisch gesteuerten Prozess nach einem der Ansprüche 7 oder 8, **dadurch gekennzeichnet, dass** das Durchflusszytometer mindestens ein Messsystem und einen Detektor und eine Verstärkungseinheit umfasst, wobei das Durchflusszytometer mit der Steuerungseinheit verbunden ist und Messsignale zur Analyse der übertragenen Signale an diese überträgt.

10. Verfahren für einen industriellen Bioreaktor (1) mit einem doppelten, zyklisch gesteuerten Prozess nach Anspruch 9, **dadurch gekennzeichnet, dass** das Messsystem die Impedanz oder die Leitfähigkeit unter Verwendung von optischen Systemen misst, welche Lichtsignale aussenden.

11. Verfahren für einen industriellen Bioreaktor (1) mit einem doppelten, zyklisch gesteuerten Prozess nach einem der Ansprüche 9 oder 10, **dadurch gekennzeichnet, dass** der Detektor ein Analog-Digital-Wandler-System (ADC) umfasst, das analoge Messungen von Vorwärtsstreulicht (FSC), Seitenstreulicht (SSC) und farbstoffspezifischen Fluoreszenzsignalen in digitale Signale umwandelt, welche von der Steuerungseinheit verarbeitet werden können.

12. Verfahren für einen industriellen Bioreaktor (1) mit einem doppelten, zyklisch gesteuerten Prozess nach Anspruch 9 bis 11, **dadurch gekennzeichnet, dass** die Verstärkungseinheit linear oder logarithmisch ausgeführt ist.

13. Verfahren für einen industriellen Bioreaktor (1) mit einem doppelten, zyklisch gesteuerten Prozess nach einem der Ansprüche 1 bis 12, **dadurch gekennzeichnet, dass** die Behandlungsleistung optimiert ist, wenn in dem Bioreaktor (1) auf der Grundlage der gemessenen zweiten sensorischen Parameterwerte eine Beschleunigung des Kultivierungsprozesses (21) und/oder ein angestrebtes biologisches Wachstum gemessen wird.

14. Verfahren für einen industriellen Bioreaktor (1) mit einem doppelten, zyklisch gesteuerten Prozess nach einem der Ansprüche 1 bis 13, **dadurch gekennzeichnet, dass** der Bioreaktor (1) als Fermenter und/oder als Keimbehälter bei Anwendungen zur Saatgutkeimung ausgeführt ist.

15. Verfahren für einen industriellen Bioreaktor (1) mit einem doppelten, zyklisch gesteuerten Prozess nach einem der Ansprüche 1 bis 14, **dadurch gekennzeichnet, dass** die Steuerungseinheit eine auf maschinellem Lernen oder künstlicher Intelligenz basierende Einheit umfasst, welche die mittels eines dielektrischen Spektroskopiesystems gemessene Behandlungsleistung und/oder die mittels des Durchflusszytometers gemessene Behandlungsleistung erfasst, wobei die ersten Betriebsparameter und/oder die zweiten Betriebsparameter von der Steuerungseinheit (13) automatisch angepasst werden, wobei zumindest die ersten und zweiten sensorischen Parameter als Eingabewerte bei der auf maschinellem Lernen oder künstlicher Intelligenz basierenden Einheit eingesetzt werden, und wobei die Ausgabe der auf maschinellem Lernen oder künstlicher Intelligenz basierenden Einheit die Anpassung der ersten Betriebsparameter und/oder der zweiten Betriebsparameter auslöst, bis die angestrebte Kultivierungsleistung mit dem angewandten Behandlungsverfahren (32) erreicht ist.

## Revendications

1. Procédé pour un bioréacteur industriel (1) comportant un processus contrôlé par un double cycle, fournissant un processus de culture (21) pour des cultures cellulaires, des composants cellulaires ou des produits métaboliques des cellules dans un milieu nutritif (11), le bioréacteur comprenant une cuve de réacteur (12) fournissant des conditions de bioréacteur contrôlées pour le processus de culture (21), et une unité de commande reliée à des dispositifs de détection (14) mesurant des valeurs de paramètres de détection (141) comprenant au moins des mesures relatives à la composition (1411) du milieu nutritif (11) et/ou à la concentration (1412) du milieu nutritif (11) et/ou à l'oxygène (1413) et/ou à la température (1414) et/ou à la valeur du pH (1415) et/ou à la stérilité (1416), et les transmettant à l'unité de commande, dans lequel l'unité de commande contrôle et/ou pilote le processus de culture (21) en ajustant les paramètres de fonctionnement du bioréacteur (1) influant sur les valeurs de paramètres de détection mesurés (141), le processus contrôlé par un double cycle étant **caractérisé par** les étapes consistant à

> ajuster, au moyen d'un cycle d'optimisation de la culture (2), l'optimisation des performances de culture (241) du processus de culture (21), des premiers paramètres de fonctionnement du bioréacteur (1) en identifiant une fenêtre biologiquement optimisée pour la culture et/ou le traitement, et en optimisant, au moyen d'un cycle d'optimisation du traitement (3), un processus de traitement (32) appliqué à la culture cellulaire pendant le processus de culture (21) au sein de la fenêtre de traitement optimisée en appliquant des deuxièmes paramètres de fonctionnement du bioréacteur (1),
>
> mesurer (22) des premiers paramètres de détection à l'aide de premiers dispositifs de détection parmi les dispositifs de détection (14), dans le cycle d'optimisation de la culture (2) (comme précédemment) en capturant la performance de culture du processus de culture (21), dans lequel les premiers paramètres de détection sont transmis à un premier analyseur (23) qui effectue un rapprochement entre la performance de culture cible et la performance de culture mesuré et, si la performance de culture cible n'est pas atteinte, les premiers paramètres opérationnels sont ajustés et les opérations de mesure (22) et de comparaison (24) sont répétées,
>
> mesurer la bioimpédance à l'aide d'un dispositif de mesure parmi les premiers dispositifs de détection mesurant la performance de culture du processus de culture (21) au moyen des premiers paramètres de détection en détectant leur réponse à une excitation électrique, grâce à quoi, au moyen d'électrodes, un signal d'excitation basé sur le courant ou le potentiel est appliqué à la culture cellulaire et la réponse est mesurée en convertissant la charge en charge ionique et vice versa, ce qui permet de détecter au moins le nombre de cellules et/ou la taille des cellules et/ou la viabilité des cellules, et
>
> déclencher, si la performance de culture cible est atteinte, le cycle d'optimisation du traitement (3), le cycle d'optimisation du traitement (3) comprenant la mesure (32) de deuxièmes paramètres de détection par des deuxièmes dispositifs de détection parmi les dispositifs de détection (14), capturant la performance de traitement du processus de traitement (31) en mesurant l'écart induit par le traitement dans la performance de culture, dans lequel les deuxièmes paramètres de détection sont transmis à un second analyseur (33) qui effectue un rapprochement entre la performance de traitement cible et la performance de traitement mesurée et, si la performance de traitement cible n'est pas atteinte, les deuxièmes paramètres opérationnels sont ajustés et la mesure (32) ainsi que le rapprochement (34) sont répétés ; dans le cas contraire, le processus d'optimisation à double cycle est achevé.

2. Procédé pour un bioréacteur industriel (1) comportant un processus contrôlé par un double cycle selon la revendication 1, **caractérisé en ce qu'**un angle de phase de la culture cellulaire est mesuré par la mesure de bioimpédance (22), cet angle de phase étant corrélé à la viabilité cellulaire, le traitement (31) étant appliqué lorsque l'angle de phase mesuré augmente, tandis que le traitement (31) est interrompu lorsque l'angle de phase mesuré diminue.

3. Procédé pour un bioréacteur industriel (1) comportant un processus contrôlé par un double cycle selon l'une quelconque des revendications 1 ou 2, **caractérisé en ce que** le processus de traitement (31) comprend l'application de champs électriques pulsés de l'ordre de la nanoseconde (nsPEF) à la culture cellulaire à l'aide d'au moins deux électrodes appliquées, les champs électriques étant appliqués en couplant une électrode à une tension plus élevée et une électrode à la masse ou à une tension plus faible, et les champs électriques pulsés ayant une forme et/ou une fréquence et/ou une intensité définissables.

4. Procédé pour un bioréacteur industriel (1) comportant un processus contrôlé par un double cycle selon la revendication 3, **caractérisé en ce que** l'unité de commande comprend des réglages de base prédéfinis pour les champs électriques pulsés de l'ordre de la nanoseconde pour chaque type de cellule possible.

5. Procédé pour un bioréacteur industriel (1) comportant un processus contrôlé par un double cycle selon l'une quelconque des revendications 1 à 4, **caractérisé en ce que** la performance de traitement est mesurée au moyen d'un système de spectroscopie diélectrique mesurant les propriétés diélectriques des cellules en fonction de la fréquence, dans lequel la permittivité dépendante de la fréquence dans une plage cible est mesurée, et dans lequel l'amplitude ou l'intensité du signal mesurée sert de valeur de paramètre cible mesurée pour la performance du traitement.

6. Procédé pour un bioréacteur industriel (1) comportant un processus contrôlé par un double cycle selon la revendication 5, **caractérisé en ce que** la plage cible est comprise entre 0,1 et 30 MHz.

7. Procédé pour un bioréacteur industriel (1) comportant un processus contrôlé par un double cycle, selon l'une quelconque des revendications 3 à 6, **caractérisé en ce que** la performance du traitement est mesurée au moyen d'un cytomètre en flux, l'un au moins des deuxièmes dispositifs de détection mesurant l'activité métabolique sur la base d'un test de fluorescence, d'une conversion d'un colorant fluorescent et d'une intensité de signal servant de valeur de paramètre cible mesurée pour la performance du traitement.

8. Procédé pour un bioréacteur industriel (1) comportant un processus contrôlé par un double cycle selon la revendication 7, **caractérisé en ce que** le test de fluorescence est le diacétate de fluorescéine (FDA).

9. Procédé pour un bioréacteur industriel (1) comportant un processus contrôlé par un double cycle selon l'une quelconque des revendications 7 ou 8, **caractérisé en ce que** le cytomètre en flux comprend au moins un système de mesure, et un détecteur et une unité d'amplification, le cytomètre en flux étant relié à l'unité de commande et transmettant des signaux de mesure à l'unité de commande en vue de l'analyse des signaux transmis.

10. Procédé pour un bioréacteur industriel (1) comportant un processus contrôlé par un double cycle selon la revendication 9, **caractérisé en ce que** le système de mesure mesure l'impédance ou la conductivité à l'aide de systèmes optiques émettant des signaux lumineux.

11. Procédé pour un bioréacteur industriel (1) comportant un processus contrôlé par un double cycle selon l'une quelconque des revendications 9 ou 10, **caractérisé en ce que** le détecteur comprend un système de conversion analogique-numérique (ADC) qui convertit les mesures analogiques de la lumière diffusée vers l'avant (FSC), de la lumière diffusée latéralement (SSC) et des signaux de fluorescence spécifiques au colorant en signaux numériques pouvant être traités par l'unité de commande.

12. Procédé pour un bioréacteur industriel (1) comportant un processus contrôlé par un double cycle selon l'une quelconque des revendications 9 à 11, **carac-**

**térisé en ce que** l'unité d'amplification est réalisée de manière linéaire ou logarithmique.

13. Procédé pour un bioréacteur industriel (1) comportant un processus contrôlé par un double cycle selon l'une quelconque des revendications 1 à 12, **caractérisé en ce que** la performance de traitement est optimisée si une accélération du processus de culture (21) et/ou une croissance biologique ciblée est mesurée dans le bioréacteur (1) sur la base des valeurs mesurées du deuxième paramètre de détection.

14. Procédé pour un bioréacteur industriel (1) comportant un processus contrôlé par un double cycle selon l'une quelconque des revendications 1 à 13, **caractérisé en ce que** le bioréacteur (1) est réalisé sous la forme d'un fermenteur et/ou d'une chambre de germination dans le cas d'applications de germination de semences.

15. Procédé pour un bioréacteur industriel (1) comportant un processus contrôlé par un double cycle selon l'une quelconque des revendications 1 à 14, **caractérisé en ce que** l'unité de commande comprend une unité basée sur l'apprentissage automatique ou l'intelligence artificielle qui capture la performance de traitement mesurée au moyen d'un système de spectroscopie diélectrique et/ou la performance de traitement mesurée au moyen du cytomètre en flux, dans lequel les premiers paramètres opérationnels et/ou les deuxièmes paramètres opérationnels sont automatiquement adaptés par l'unité de commande (13), dans lequel au moins les premiers et deuxièmes paramètres de détection sont appliqués en tant que valeurs d'entrée à l'unité basée sur l'apprentissage automatique ou l'intelligence artificielle, et dans lequel la sortie de l'unité basée sur l'apprentissage automatique ou l'intelligence artificielle déclenche le réglage des premiers paramètres opérationnels et/ou des deuxièmes paramètres opérationnels jusqu'à ce que la performance de culture cible avec le processus de traitement appliqué (32) soit atteinte.

Fig. 1

Fig. 2

R=1/G

V_rest

"equivalent circuit"

Membrane

Fig. 3

Fig. 4

Fig. 5

Fig. 6

**Fig. 7**

FCM – Metabolic activity
(cellular FDA cleavage)

Fig. 8

FCM - Metabolic activity
(cellular FDA cleavage)

Mean cellular fluorescence intensity (FIU)

Kinetic cycle (measurement-Nr)

**Fig. 9**

33

Fig. 10

**Fig. 11**

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2725095 B1
- WO 2016092281 A1
- CN 105044038 A
- US 10751715 B1
- US 6911201 B
- US 20100233130 A
- WO 2008152640 A
- US 9127242 B
- CN 105543085 A
- US 10336978 B2
- EP 3819367 A1